# EUROPEAN PATENT APPLICATION

(11) **EP 4 083 229 A1**
(43) Date of publication of application: **02.11.2022**
(21) Application number: 22164769.6
(22) Date of filing: 15.10.2018
(51) Int. Cl.: C12Q 1/686, G01N 21/64, G01N 33/58, G01N 33/533, C12Q 1/70, C12Q 1/6818, C12Q 1/6823

(54) **METHODS AND COMPOSITIONS FOR NUCLEIC ACID DETECTION**

(30) Priority: 16.10.2017 US 201762573046 P; 08.02.2018 US 201815892245
(62) Divisional of application: 18867832.0
(71) Applicant: Chromacode, Inc., Carlsbad, California 92008 (US)
(72) Inventor: RAJAGOPAL, Aditya, Carlsbad, CA 92011 (US); MACDONALD, Christopher, Carlsbad, CA 92011 (US)
(74) Representative: HGF

(57) **Abstract**

The present disclosure provides methods and compositions for nucleic acid detection. Nucleic acids may be derived from any source including, for example, viruses, bacterial cells, and eukaryotic cells. The methods of the present disclosure may be used to detect the presence of both a first and second member of a plurality of target nucleic acids in a sample, by the generation of one signal using polymerase chain reaction, a signal oligonucleotide probe and a template.

## Description

### CROSS-REFERENCE

This application is a continuation of U.S. Patent Application No. 15/892,245, filed on February 8, 2018; and claims the benefit of U.S. Provisional Patent Application No. 62/573,046, filed on October 16, 2017; which applications are incorporated herein by reference in their entirety.

### BACKGROUND

Real-Time Polymerase Chain Reaction (PCR) is a process of monitoring a PCR reaction by recording the fluorescence generated either by an intercalating dye such as SYBR Green or a reporter probe at each cycle. This is generally performed on a Real-Time PCR instrument that executes thermal cycling of the sample to complete the PCR cycles and at a specified point in each cycle measures the fluorescence of the sample in each channel through a series of excitation/emission filter sets.

### SUMMARY

Quantitative real-time PCR (qPCR) is a useful technique for measuring a limited number of target analytes in a sample. Current limitations of standard qPCR instruments lie in the inability to accurately measure distinct analytes in a single reaction. Multiplex qPCR technology allows for a limited number of analytes to be detected in a reaction using individual fluorescent reporters. Additional analytes can be measured by the use of identical fluorescent reporters at different concentrations, however the requirements of deconvolution of this fluorescent signal keeps the number of analytes that can be measured in a single reaction to a minimum. Therefore, a need remains for a method of detecting the presence of any subset of a large number of target analytes from a single sample.

Disclosed herein are methods and compositions for detection of one or more nucleic acid analytes from a sample. In some aspects, one or more subsets of a large number of target analytes from a single sample may be detected using a single fluorescent signal. This is a key improvement over current multiplexing technologies.

In an aspect, the present disclosure provides a method of detecting the presence or absence of at least one member of a plurality of target nucleic acids in a sample, the method comprising: (a) providing a sample comprising, or potentially comprising, at least one member of the plurality of target nucleic acids; (b) forming a mixture comprising the sample and (i) a signal oligonucleotide probe; (ii) a first forward oligonucleotide primer comprising: a first region complementary to a first region of a first member of the plurality of target nucleic acids; and a second region complementary or homologous to the signal oligonucleotide probe; (iii) a first reverse oligonucleotide primer comprising a region complementary to a second region of the first member of the plurality of target nucleic acids; (iv) a second forward oligonucleotide primer comprising: a first region complementary to a first region of a second member of the plurality of target nucleic acids; and a second region complementary or homologous to the signal oligonucleotide probe; and (v) a second reverse oligonucleotide primer comprising a region complementary to a second region of the second member of the plurality of target nucleic acids; (c) thermally cycling the mixture under conditions appropriate to amplify each member of the plurality of target nucleic acids with polymerase chain reaction (PCR), such that the signal oligonucleotide probe is degraded and a signal is generated if at least one member of the plurality of target nucleic acids is present in the mixture; and (d) detecting the presence or absence of the signal. In some embodiments, one or both of the second region of the first oligonucleotide primer and the second region of the second forward oligonucleotide primer are complementary to the signal oligonucleotide probe. In some embodiments, at least one of the second region of the first oligonucleotide primer and the second region of the second forward oligonucleotide primer are homologous to the signal oligonucleotide probe. In some embodiments, the mixture further comprises a universal oligonucleotide primer. In some embodiments, the first forward oligonucleotide primer and the second forward oligonucleotide primer each further comprise a third region complementary to the universal oligonucleotide primer. In some embodiments, the mixture further comprises a nucleic acid enzyme. In some embodiments, the signal oligonucleotide probe is degraded by the nucleic acid enzyme. In some embodiments, the nucleic acid enzyme has endonuclease activity. In some embodiments, the nucleic acid enzyme has exonuclease activity. In some embodiments, the signal oligonucleotide probe is degraded by the exonuclease activity of the nucleic acid enzyme. In some embodiments, the nucleic acid enzyme is a deoxyribonucleic acid polymerase. In some embodiments, the signal oligonucleotide probe comprises a signal tag. In some embodiments, in (c), the signal is generated by the signal tag. In some embodiments, the signal tag generates the signal upon degradation of the signal oligonucleotide probe. In some embodiments, the first region of the first forward oligonucleotide primer is 5' relative to the second region of the first forward oligonucleotide primer. In some embodiments, in (c), each thermal cycle is performed at an annealing temperature appropriate for annealing of the first region of the first forward oligonucleotide primer to the first target nucleic acid and annealing of the second region of the first forward oligonucleotide primer to the signal oligonucleotide probe. In some embodiments, the first region of the second forward oligonucleotide primer is 5' relative to the second region of the second forward oligonucleotide primers. In some embodiments, the second region of the first forward oligonucleotide primer has between about 50% and about 100%, between about 60% and about 100%, between about 70% and about 100%, between about 80% and about 100%, between about 90% and about 100%, between about 95% and about 100%, between about 99% and about 100%, or about 100% identity with the second region of the second forward oligonucleotide primer. In some embodiments, in (c), each thermal cycle is performed at an annealing temperature appropriate for annealing of the first region of the second forward oligonucleotide primer to the second target nucleic acid and annealing of the second region of the second forward oligonucleotide primer to the signal oligonucleotide probe. In some embodiments, the method further comprises correlating the presence of the signal to the presence of at least one member of the plurality of target nucleic acids or correlating the absence of the signal with the absence of each member of the plurality of target nucleic acids.

In an aspect, the present disclosure provides a method of detecting the presence or absence of at least one member of a plurality of target nucleic acids in a sample, the method comprising: (a) providing a sample comprising, or potentially comprising, at least one member of the plurality of target nucleic acids; (b) forming a mixture of the sample and (i) a signal oligonucleotide probe; (ii) a first forward oligonucleotide primer comprising a region with complementarity to a first member of the plurality of target nucleic acids; (iii) a first reverse oligonucleotide primer comprising a region with complementarity to the first member of the plurality of target nucleic acids; (iv) a second forward oligonucleotide primer comprising a region with complementarity to a second member of the plurality of target nucleic acids; and (v) a second reverse oligonucleotide primer comprising a region with complementarity to the second member of the plurality of target nucleic acids; (c) thermally cycling the mixture under conditions appropriate to amplify each member of the plurality of target nucleic acids with polymerase chain reaction (PCR) and generating a signal if at least one member of the plurality of target nucleic acids is present in the mixture; and (d) detecting the presence or absence of the signal. In some embodiments, the mixture comprises a plurality of the first and second forward oligonucleotide primers and a plurality of the first and second oligonucleotide reverse primers. In some embodiments, the mixture further comprises a nucleic acid enzyme. In some embodiments, the signal oligonucleotide probe is degraded by the nucleic acid enzyme. In some embodiments, the signal oligonucleotide probe comprises a signal tag. In some embodiments, in (c), the signal is generated by the signal tag. In some embodiments, the signal tag generates the signal upon degradation of the signal oligonucleotide probe. In some embodiments, the region of the first forward oligonucleotide primer has complementarity to a first region of the first member of the plurality of target nucleic acids. In some embodiments, the region of the second forward oligonucleotide primer has complementarity to a second region of the first member of the plurality of target nucleic acids. In some embodiments, the first forward oligonucleotide primer further comprises an additional region with complementarity or homology to the signal oligonucleotide probe. In some embodiments, the additional region of the first forward oligonucleotide primer has complementarity to the signal oligonucleotide probe. In some embodiments, the additional region of the first forward oligonucleotide primer has homology to the signal oligonucleotide probe. In some embodiments, the region of the first forward oligonucleotide primer is 5' relative to the additional region of the first forward oligonucleotide primers. In some embodiments, the additional region of the first forward oligonucleotide primer has between about 50% and about 100% complementarity to the signal oligonucleotide probe. In some embodiments, the additional region of the first forward oligonucleotide primer has between about 60% and about 100% complementarity to the signal oligonucleotide probe. In some embodiments, the additional region of the first forward oligonucleotide primer has between about 70% and about 100% complementarity to the signal oligonucleotide probe. In some embodiments, the additional region of the first forward oligonucleotide primer has between about 80% and about 100% complementarity to the signal oligonucleotide probe. In some embodiments, the additional region of the first forward oligonucleotide primer has between about 90% and about 100% complementarity to the signal oligonucleotide probe. In some embodiments, the additional region of the first forward oligonucleotide primer has between about 95% and about 100% complementarity to the signal oligonucleotide probe. In some embodiments, the additional region of the first forward oligonucleotide primer has between about 99% and about 100% complementarity to the signal oligonucleotide probe. In some embodiments, the additional region of the first forward oligonucleotide primer has about 100% complementarity to the signal oligonucleotide probe. In some embodiments, the additional region of the first forward oligonucleotide primer has length of between about 5 nucleotides and about 500 nucleotides. In some embodiments, the additional region of the first forward oligonucleotide primer has length of between about 5 nucleotides and about 300 nucleotides. In some embodiments, the additional region of the first forward oligonucleotide primer has length of between about 5 nucleotides and about 100 nucleotides. In some embodiments, the additional region of the first forward oligonucleotide primer has length of between about 5 nucleotides and about 50 nucleotides. In some embodiments, the additional region of the first forward oligonucleotide primer has length of between about 5 nucleotides and about 30 nucleotides. In some embodiments, the additional region of the first forward oligonucleotide primer has length of between about 5 nucleotides and about 20 nucleotides. In some embodiments, the additional region of the first forward oligonucleotide primer has length of between about 5 nucleotides and about 15 nucleotides. In some embodiments, in (c), each thermal cycle is performed at an annealing temperature appropriate for annealing of the region of the first forward oligonucleotide primer to the first member of the plurality of target nucleic acids and annealing of the additional region of the first forward oligonucleotide primer to the signal oligonucleotide probe. In some embodiments, the second forward oligonucleotide primer further comprises an additional region with complementarity or homology to the signal oligonucleotide probe. In some embodiments, the additional region of the second forward oligonucleotide primer has complementarity to the signal oligonucleotide probe. In some embodiments, the additional region of the second forward oligonucleotide primer has homology to the signal oligonucleotide probe. In some embodiments, the region of the second forward oligonucleotide primer is 5' relative to the additional region of the second forward oligonucleotide primers. In some embodiments, the additional region of the second forward oligonucleotide primer has between about 50% and about 100% complementarity to the signal oligonucleotide probe. In some embodiments, the additional region of the second forward oligonucleotide primer has between about 60% and about 100% complementarity to the signal oligonucleotide probe. In some embodiments, the additional region of the second forward oligonucleotide primer has between about 70% and about 100% complementarity to the signal oligonucleotide probe. In some embodiments, the additional region of the second forward oligonucleotide primer has between about 80% and about 100% complementarity to the signal oligonucleotide probe. In some embodiments, the additional region of the second forward oligonucleotide primer has between about 90% and about 100% complementarity to the signal oligonucleotide probe. In some embodiments, the additional region of the second forward oligonucleotide primer has between about 95% and about 100% complementarity to the signal oligonucleotide probe. In some embodiments, the additional region of the second forward oligonucleotide primer has between about 99% and about 100% complementarity to the signal oligonucleotide probe. In some embodiments, the additional region of the second forward oligonucleotide primer has about 100% complementarity to the signal oligonucleotide probe. In some embodiments, the additional region of the first forward oligonucleotide primer has between about 50% and about 100% identity with the additional region of the second forward oligonucleotide primer. In some embodiments, the additional region of the second forward oligonucleotide primer has length of between about 5 nucleotides and about 500 nucleotides. In some embodiments, the additional region of the second forward oligonucleotide primer has length of between about 5 nucleotides and about 300 nucleotides. In some embodiments, the additional region of the second forward oligonucleotide primer has length of between about 5 nucleotides and about 100 nucleotides. In some embodiments, the additional region of the second forward oligonucleotide primer has length of between about 5 nucleotides and about 50 nucleotides. In some embodiments, the additional region of the second forward oligonucleotide primer has length of between about 5 nucleotides and about 30 nucleotides. In some embodiments, the additional region of the second forward oligonucleotide primer has length of between about 5 nucleotides and about 20 nucleotides. In some embodiments, the additional region of the second forward oligonucleotide primer has length of between about 5 nucleotides and about 15 nucleotides. In some embodiments, the additional region of the first forward oligonucleotide primer has between about 50% and about 100% identity with the additional region of the second forward oligonucleotide primer. In some embodiments, the additional region of the first forward oligonucleotide primer has between about 60% and about 100% identity with the additional region of the second forward oligonucleotide primer. In some embodiments, the additional region of the first forward oligonucleotide primer has between about 70% and about 100% identity with the additional region of the second forward oligonucleotide primer. In some embodiments, the additional region of the first forward oligonucleotide primer has between about 80% and about 100% identity with the additional region of the second forward oligonucleotide primer. In some embodiments, the additional region of the first forward oligonucleotide primer has between about 90% and about 100% identity with the additional region of the second forward oligonucleotide primer. In some embodiments, the additional region of the first forward oligonucleotide primer has between about 95% and about 100% identity with the additional region of the second forward oligonucleotide primer. In some embodiments, the additional region of the first forward oligonucleotide primer has between about 99% and about 100% identity with the additional region of the second forward oligonucleotide primer. In some embodiments, the additional region of the first forward oligonucleotide primer has about 100% identity with the additional region of the second forward oligonucleotide primer. In some embodiments, in (c), each thermal cycle is performed at an annealing temperature appropriate for annealing of the first region of the second forward oligonucleotide primer to the second member of the plurality of target nucleic acids and annealing of the additional region of the second forward oligonucleotide primer to the signal oligonucleotide probe. In some embodiments, the nucleic acid enzyme is a nucleic acid polymerase. In some embodiments, the nucleic acid enzyme has exonuclease activity. In some embodiments, the signal oligonucleotide probe is degraded by the exonuclease activity of the nucleic acid enzyme. In some embodiments, the nucleic acid enzyme has endonuclease activity. In some embodiments, the method further comprises correlating the absence of the signal with the absence of each member of the plurality of target nucleic acids. In some embodiments, the signal oligonucleotide probe does not have complementarity to any member of the plurality of target nucleic acids. In some embodiments, the method further comprises correlating the presence of the signal to the presence of at least one member of the plurality of target nucleic acids. In some embodiments, the nucleic acid enzyme is a deoxyribonucleic acid polymerase. In some embodiments, the deoxyribonucleic acid polymerase is a Taq polymerase. In some embodiments, the one or more target nucleic acids are deoxyribonucleic acids. In some embodiments, the one or more target nucleic acids are ribonucleic acids. In some embodiments, the signal is a fluorescent signal. In some embodiments, the signal tag comprises a fluorophore and a quencher. In some embodiments, the fluorophore is FAM, TET, HEX, JOE, Cy3, Cy5, or any combination thereof. In some embodiments, the mixture further comprises a universal oligonucleotide primer. In some embodiments, the first forward oligonucleotide primer further comprises a region with complementarity or homology to the universal oligonucleotide primer. In some embodiments, the second forward oligonucleotide primer further comprises a region with complementarity or homology to the universal oligonucleotide primer. In some embodiments, the mixture formed at (b) further comprises: an nth forward oligonucleotide primer comprising: a first region with complementarity to an nth member of the plurality of target nucleic acids; and an nth reverse oligonucleotide primer comprising a region with complementarity to a region of an nth member of the plurality of target nucleic acids; wherein n is an integer greater than 2. In some embodiments, the mixture comprises a plurality of the nth forward and nth reverse oligonucleotide primers. In some embodiments, the region of the nth forward oligonucleotide primer has complementarity to a first region of the nth member of the plurality of target nucleic acids. In some embodiments, the region of the nth reverse oligonucleotide primer has complementarity to a second region of the nth member of the plurality of target nucleic acids. In some embodiments, the nth forward oligonucleotide primer further comprises an additional region with complementarity to the signal oligonucleotide probe. In some embodiments, the region of the nth forward oligonucleotide primer is 5' relative to the additional region of the nth forward oligonucleotide primer. In some embodiments; the mixture formed at (b) further comprises: an xth signal oligonucleotide probe; a yth forward oligonucleotide primer comprising a first region with complementarity to a yth member of the plurality of target nucleic acids; and a yth reverse oligonucleotide primer comprising a region with complementarity to a second region of the yth member of the plurality of target nucleic acids; in (c), the xth signal oligonucleotide probe is degraded by the nucleic acid polymerase such that an xth signal is generated if at least one of the yth member of the plurality of target nucleic acids is present in the mixture; (e) further comprises correlating the presence of the xth signal to the presence of at least one of the yth members of the plurality target nucleic acids in the sample; and x is an integer greater than 1 and y is an integer greater than n. In some embodiments, the xth signal oligonucleotide probe comprises an xth signal tag. In some embodiments, the mixture comprises a plurality of the yth forward and yth reverse oligonucleotide primers. In some embodiments, the region of the yth forward oligonucleotide primer has complementarity to a first region of the yth member of the plurality of target nucleic acids. In some embodiments, the region of the yth reverse oligonucleotide primer has complementarity to a second region of the yth member of the plurality of target nucleic acids. In some embodiments, the yth forward oligonucleotide primer further comprises an additional region with complementarity to the signal oligonucleotide probe. In some embodiments, the first region of each of the plurality of yth forward oligonucleotide primers is 5' relative to the additional region of each of the plurality of yth forward oligonucleotide primers. In some embodiments, the signal tags are different. In some embodiments, the signal tags are the same. In some embodiments, the signals are different signal types. In some embodiments, the signals are different intensities of the same signal type.

In an aspect, the present disclosure provides a kit for detecting the presence or absence of at least one member of a plurality of target nucleic acids in a sample, comprising: (a) a signal oligonucleotide probe; (b) a first forward oligonucleotide primer comprising: a first region with complementarity to a first member of the plurality of target nucleic acids; and a second region with complementarity or homology to the signal oligonucleotide probe; (c) a first reverse oligonucleotide primer comprising a region with complementarity to the first member of the plurality of target nucleic acids; (d) a second forward oligonucleotide primer comprising: a first region with complementarity to a second member of the plurality of target nucleic acids; and a second region with complementarity or homology to the signal oligonucleotide probe; and (e) a second reverse oligonucleotide primer comprising a region with complementarity to the second member of the plurality of target nucleic acids. In some embodiments, the kit comprises a plurality of the first and second forward oligonucleotide primers and a plurality of the first and second oligonucleotide reverse primers. In some embodiments, the signal oligonucleotide probe comprises a signal tag. In some embodiments, the second region of the first forward oligonucleotide primer has complementarity to the signal oligonucleotide probe. In some embodiments, the second region of the first forward oligonucleotide primer has homology to the signal oligonucleotide probe. In some embodiments, the second region of the second forward oligonucleotide primer has complementarity to the signal oligonucleotide probe. In some embodiments, the second region of the second forward oligonucleotide primer has homology to the signal oligonucleotide probe. In some embodiments, the kit further comprises a universal oligonucleotide primer. In some embodiments, the first forward oligonucleotide primer further comprises a region with complementarity or homology to the universal oligonucleotide primer. In some embodiments, the first forward oligonucleotide primer further comprises a region with complementarity or homology to the universal oligonucleotide primer. In some embodiments, the kit further comprises: a plurality of an nth forward oligonucleotide primer comprising: a first region with complementarity to a first region of an nth member of the plurality of target nucleic acids; and a second region with complementarity to the signal oligonucleotide probe; and a plurality of an nth reverse oligonucleotide primer comprising a region with complementarity to a second region of an nth member of the plurality of target nucleic acids; wherein n is an integer greater than 2. In some embodiments, the kit further comprises: an xth signal oligonucleotide probe comprising an xth signal tag; a plurality of a yth forward oligonucleotide primer comprising: a first region with complementarity to a first region of a yth member of the plurality of target nucleic acids; and a second region with complementarity to the xth signal oligonucleotide probe; and a plurality of a yth reverse oligonucleotide primer comprising a region with complementarity to a second region of the yth member of the plurality of target nucleic acids; wherein x is an integer greater than 1 and y is an integer greater than n. In some embodiments, the signal tags are different. In some embodiments, the signal tags are the same. In some embodiments, the signal oligonucleotide probes are present in different concentrations. In some embodiments, at least one of the oligonucleotide primers is lyophilized. In some embodiments, each of the oligonucleotide primers is lyophilized. In some embodiments, the signal oligonucleotide probe is lyophilized. In some embodiments, the signal tag comprises a fluorophore and a quencher. In some embodiments, the fluorophore is FAM, TET, HEX, JOE, Cy3, Cy5, or any combination thereof. In some embodiments, the second region of the first forward oligonucleotide primer has between about 50% and about 100% complementarity to the signal oligonucleotide probe. In some embodiments, the second region of the first forward oligonucleotide primer has between about 60% and about 100% complementarity to the signal oligonucleotide probe. In some embodiments, the second region of the first forward oligonucleotide primer has between about 70% and about 100% complementarity to the signal oligonucleotide probe. In some embodiments, the second region of the first forward oligonucleotide primer has between about 80% and about 100% complementarity to the signal oligonucleotide probe. In some embodiments, the second region of the first forward oligonucleotide primer has between about 90% and about 100% complementarity to the signal oligonucleotide probe. In some embodiments, the second region of the first forward oligonucleotide primer has between about 95% and about 100% complementarity to the signal oligonucleotide probe. In some embodiments, the second region of the first forward oligonucleotide primer has between about 99% and about 100% complementarity to the signal oligonucleotide probe. In some embodiments, the second region of the first forward oligonucleotide primer has about 100% complementarity to the signal oligonucleotide probe. In some embodiments, the second region of the second forward oligonucleotide primer has between about 50% and about 100% complementarity to the signal oligonucleotide probe. In some embodiments, the second region of the second forward oligonucleotide primer has between about 60% and about 100% complementarity to the signal oligonucleotide probe. In some embodiments, the second region of the second forward oligonucleotide primer has between about 70% and about 100% complementarity to the signal oligonucleotide probe. In some embodiments, the second region of the second forward oligonucleotide primer has between about 80% and about 100% complementarity to the signal oligonucleotide probe. In some embodiments, the second region of the second forward oligonucleotide primer has between about 90% and about 100% complementarity to the signal oligonucleotide probe. In some embodiments, the second region of the second forward oligonucleotide primer has between about 95% and about 100% complementarity to the signal oligonucleotide probe. In some embodiments, the second region of the second forward oligonucleotide primer has between about 99% and about 100% complementarity to the signal oligonucleotide probe. In some embodiments, the second region of the second forward oligonucleotide primer has about 100% complementarity to the signal oligonucleotide probe. In some embodiments, the second region of the first forward oligonucleotide primer has between about 50% and about 100% identity with the second region of the second forward oligonucleotide primer. In some embodiments, the second region of the first forward oligonucleotide primer has between about 50% identity with the second region of the second forward oligonucleotide primer. In some embodiments, the second region of the first forward oligonucleotide primer has between about 60% and about 100% identity with the second region of the second forward oligonucleotide primer. In some embodiments, the second region of the first forward oligonucleotide primer has between about 70% and about 100% identity with the second region of the second forward oligonucleotide primer. In some embodiments, the second region of the first forward oligonucleotide primer has between about 80% and about 100% identity with the second region of the second forward oligonucleotide primer. In some embodiments, the second region of the first forward oligonucleotide primer has between about 90% and about 100% identity with the second region of the second forward oligonucleotide primer. In some embodiments, the second region of the first forward oligonucleotide primer has between about 95% and about 100% identity with the second region of the second forward oligonucleotide primer. In some embodiments, the second region of the first forward oligonucleotide primer has between about 99% and about 100% identity with the second region of the second forward oligonucleotide primer. In some embodiments, the second region of the first forward oligonucleotide primer has about 100% identity with the second region of the second forward oligonucleotide primer. In some embodiments, the second region of the first forward oligonucleotide primer has length of between about 5 nucleotides and about 500 nucleotides. In some embodiments, the second region of the first forward oligonucleotide primer has length of between about 5 nucleotides and about 300 nucleotides. In some embodiments, the second region of the first forward oligonucleotide primer has length of between about 5 nucleotides and about 100 nucleotides. In some embodiments, the second region of the first forward oligonucleotide primer has length of between about 5 nucleotides and about 50 nucleotides. In some embodiments, the second region of the first forward oligonucleotide primer has length of between about 5 nucleotides and about 30 nucleotides. In some embodiments, the second region of the first forward oligonucleotide primer has length of between about 5 nucleotides and about 20 nucleotides. In some embodiments, the second region of the first forward oligonucleotide primer has length of between about 5 nucleotides and about 15 nucleotides. In some embodiments, the second region of the second forward oligonucleotide primer has length of between about 5 nucleotides and about 500 nucleotides. In some embodiments, the second region of the second forward oligonucleotide primer has length of between about 5 nucleotides and about 300 nucleotides. In some embodiments, the second region of the second forward oligonucleotide primer has length of between about 5 nucleotides and about 100 nucleotides. In some embodiments, the second region of the second forward oligonucleotide primer has length of between about 5 nucleotides and about 50 nucleotides. In some embodiments, the second region of the second forward oligonucleotide primer has length of between about 5 nucleotides and about 30 nucleotides. In some embodiments, the second region of the second forward oligonucleotide primer has length of between about 5 nucleotides and about 20 nucleotides. In some embodiments, the second region of the second forward oligonucleotide primer has length of between about 5 nucleotides and about 15 nucleotides.

In an aspect, the present disclosure provides a method of detecting the presence or absence of a first and second member of a plurality of target nucleic acids in a sample, the method comprising: (a) providing a sample comprising, or potentially comprising, the first and second members of the plurality of target nucleic acids; (b) forming a mixture comprising the sample and (i) a template nucleic acid; (ii) a signal oligonucleotide probe with complementarity to the template nucleic acid; (iii) a first oligonucleotide primer comprising a region with complementarity to the first member of the plurality of target nucleic acids; and (iv) a second oligonucleotide primer comprising a region with complementarity to the second member of the plurality of target nucleic acids; (c) subjecting the mixture to conditions sufficient to anneal the first oligonucleotide primer to the first member of the plurality of target nucleic acids and the second oligonucleotide primer to the second member of the plurality of target nucleic acids, such that the first and second oligonucleotide primers are degraded; (d) thermally cycling the mixture under reaction conditions appropriate to amplify the template nucleic acid with polymerase chain reaction (PCR), such that a signal is generated if both the first and second members of the plurality of target nucleic acids are present in the mixture; and (e) detecting the presence or absence of the signal. In some embodiments, the mixture comprises a plurality of the first and second oligonucleotide primers. In some embodiments, the region of the first oligonucleotide primer has complementarity to a first region of the first member of the plurality of target nucleic acids. In some embodiments, the region of the second oligonucleotide primer has complementarity to a first region of the second member of the plurality of target nucleic acids. In some embodiments, the signal oligonucleotide probe has complementarity to a first region of the template nucleic acid. In some embodiments, the first oligonucleotide primer further comprises an additional region with complementarity to a second region of the template nucleic acid. In some embodiments, the additional region of the first oligonucleotide primer comprises deoxyribonucleic acid. In some embodiments, in (c), the conditions are insufficient for annealing of the additional region of the first oligonucleotide primer to the first member of the plurality of target nucleic acids. In some embodiments, the additional region of the first oligonucleotide primer has between about 50% and about 100% complementarity to the template nucleic acid. In some embodiments, the additional region of the first oligonucleotide primer has between about 60% and about 100% complementarity to the template nucleic acid. In some embodiments, the additional region of the first oligonucleotide primer has between about 70% and about 100% complementarity to the template nucleic acid. In some embodiments, the additional region of the first oligonucleotide primer has between about 80% and about 100% complementarity to the template nucleic acid. In some embodiments, the additional region of the first oligonucleotide primer has between about 90% and about 100% complementarity to the template nucleic acid. In some embodiments, the additional region of the first oligonucleotide primer has between about 95% and about 100% complementarity to the template nucleic acid. In some embodiments, the additional region of the first oligonucleotide primer has between about 99% and about 100% complementarity to the template nucleic acid. In some embodiments, the additional region of the first oligonucleotide primer has about 100% complementarity to the template nucleic acid. In some embodiments, the additional region of the first oligonucleotide primer has length of between about 5 nucleotides and about 500 nucleotides. In some embodiments, the additional region of the first oligonucleotide primer has length of between about 5 nucleotides and about 300 nucleotides. In some embodiments, the additional region of the first oligonucleotide primer has length of between about 5 nucleotides and about 100 nucleotides. In some embodiments, the additional region of the first oligonucleotide primer has length of between about 5 nucleotides and about 50 nucleotides. In some embodiments, the additional region of the first oligonucleotide primer has length of between about 5 nucleotides and about 30 nucleotides. In some embodiments, the additional region of the first oligonucleotide primer has length of between about 5 nucleotides and about 20 nucleotides. In some embodiments, the additional region of the first oligonucleotide primer has length of between about 5 nucleotides and about 15 nucleotides. In some embodiments, the additional region of the second oligonucleotide primer has length of between about 5 nucleotides and about 500 nucleotides. In some embodiments, the second oligonucleotide primer further comprises an additional region with complementarity to a third region of the template nucleic acid. In some embodiments, the additional region of the second oligonucleotide primer comprises deoxyribonucleic acid. In some embodiments, in (c), the conditions are insufficient for annealing of the additional region of the second oligonucleotide primer to the second member of the plurality of target nucleic acids. In some embodiments, the first region of the template nucleic acid is 3' of the second region of the template nucleic acid and 5' of the third region of the template nucleic acid. In some embodiments, the additional region of the second oligonucleotide primer has between about 50% and about 100% complementarity to the template nucleic acid. In some embodiments, the additional region of the second oligonucleotide primer has between about 60% and about 100% complementarity to the template nucleic acid. In some embodiments, the additional region of the second oligonucleotide primer has between about 70% and about 100% complementarity to the template nucleic acid.

In some embodiments, the additional region of the second oligonucleotide primer has between about 80% and about 100% complementarity to the template nucleic acid. In some embodiments, the additional region of the second oligonucleotide primer has between about 90% and about 100% complementarity to the template nucleic acid. In some embodiments, the additional region of the second oligonucleotide primer has between about 95% and about 100% complementarity to the template nucleic acid. In some embodiments, the additional region of the second oligonucleotide primer has between about 99% and about 100% complementarity to the template nucleic acid. In some embodiments, the additional region of the second oligonucleotide primer has about 100% complementarity to the template nucleic acid. In some embodiments, the additional region of the first oligonucleotide primer is not identical to the additional region of the second oligonucleotide primer. In some embodiments, the additional region of the second oligonucleotide primer has length of between about 5 nucleotides and about 300 nucleotides. In some embodiments, the additional region of the second oligonucleotide primer has length of between about 5 nucleotides and about 100 nucleotides. In some embodiments, the additional region of the second oligonucleotide primer has length of between about 5 nucleotides and about 50 nucleotides. In some embodiments, the additional region of the second oligonucleotide primer has length of between about 5 nucleotides and about 30 nucleotides. In some embodiments, the additional region of the second oligonucleotide primer has length of between about 5 nucleotides and about 20 nucleotides. In some embodiments, the additional region of the second oligonucleotide primer has length of between about 5 nucleotides and about 15 nucleotides. In some embodiments, in (c), the additional region of the first oligonucleotide primer and the additional region of the second oligonucleotide primer are released. In some embodiments, the mixture further comprises one or more nucleic acid enzymes. In some embodiments, the signal oligonucleotide probe comprises a signal tag. In some embodiments, the method further comprises (f) correlating the presence of the signal to the presence of both the first and second members of the plurality of target nucleic acids in the sample. In some embodiments, in (c), the first and second oligonucleotide primers are degraded by the one or more nucleic acid enzymes. In some embodiments, in (d), the signal is generated by the signal tag. In some embodiments, the signal tag generates the signal upon degradation of the signal oligonucleotide probe. In some embodiments, the signal oligonucleotide probe is degraded by the one or more nucleic acid enzymes. In some embodiments, the mixture in (b) further comprises (vi) a third oligonucleotide primer comprising a region with complementarity to a second region of the first member of the plurality of target nucleic acids; and (vii) a fourth oligonucleotide primer comprising a region with complementarity to a second region of the second member of the plurality of target nucleic acids. In some embodiments, the mixture comprises a plurality of the third and fourth oligonucleotide primers. In some embodiments, in (c), the conditions are sufficient to denature the plurality of target nucleic acids. In some embodiments, the method further comprises in (c), thermally cycling the mixture under reaction conditions appropriate to amplify the first and second members of the plurality of target nucleic acids with polymerase chain reaction. In some embodiments, the one or more nucleic acid enzymes comprise a nucleic acid polymerase. In some embodiments, the one or more nucleic acid enzymes have exonuclease activity. In some embodiments, the signal oligonucleotide probe is degraded by the exonuclease activity of the one or more nucleic acid enzymes. In some embodiments, the one or more nucleic acid enzymes have endonuclease activity. In some embodiments, the first oligonucleotide primer and the second oligonucleotide primer are degraded by the endonuclease activity of the one or more nucleic acid enzymes. In some embodiments, the plurality of target nucleic acids comprises a plurality of deoxyribonucleic acids. In some embodiments, the plurality of target nucleic acids comprises a plurality of ribonucleic acids. In some embodiments, the plurality of ribonucleic acids comprises a plurality of messenger ribonucleic acids. In some embodiments, the template nucleic acid is a deoxyribonucleic acid. In some embodiments, the template nucleic acid is a ribonucleic acid. In some embodiments, the one or more nucleic acid enzymes have RNase activity. In some embodiments, the first oligonucleotide primer and the second oligonucleotide primer are degraded by RNase activity of the one or more nucleic acid enzymes. In some embodiments, the one or more nucleic acid enzymes comprise an RNase H. In some embodiments, the one or more nucleic acid enzymes comprise an RNase III. In some embodiments, the RNase III is Dicer. In some embodiments, the one or more nucleic acid enzymes comprise an endonuclease I. In some embodiments, the endonuclease I is T7 endonuclease I. In some embodiments, the one or more nucleic acid enzymes are capable of degrading a nucleic acid comprising a non-natural nucleotide. In some embodiments, the one or more nucleic acid enzymes comprise an endonuclease V. In some embodiments, the endonuclease V is E. coli endonuclease V. In some embodiments, the region of the first oligonucleotide primer comprises one or more ribonucleotide bases. In some embodiments, the region of the second oligonucleotide primer comprises one or more ribonucleotide bases. In some embodiments, the first oligonucleotide primer further comprises at least one non-natural nucleotide. In some embodiments, the non-natural nucleotide is deoxyinosine. In some embodiments, the second oligonucleotide primer further comprises at least one non-natural nucleotide. In some embodiments, the non-natural nucleotide is deoxyinosine. In some embodiments, the signal oligonucleotide probe does not have complementarity to the any of the plurality of target nucleic acids. In some embodiments, the signal tag comprises a fluorophore and a quencher. In some embodiments, the fluorophore is FAM, TET, HEX, JOE, Cy3, Cy5, or any combination thereof. In some embodiments, the method further comprises correlating the absence of the signal with the absence of both the first and second member of the plurality of target nucleic acids.

In an aspect, the present disclosure provides a method of detecting the presence or absence of a first and second member of a plurality of target nucleic acids in a sample, the method comprising: (a) providing a sample comprising, or potentially comprising, the first and second members of the plurality of target nucleic acids (b) forming a mixture comprising the sample and (i) a template nucleic acid; (ii) a signal oligonucleotide probe with complementarity to the template nucleic acid; (iii) a first forward oligonucleotide primer comprising a region with complementarity to the first member of the plurality of target nucleic acids; (iv) a first reverse oligonucleotide primer comprising a region with complementarity the first member of the plurality of target nucleic acids; (v) a second forward oligonucleotide primer comprising a first region with complementarity to the second member of the plurality of target nucleic acids; and (vi) a second reverse oligonucleotide primer comprising a region with complementarity to the second member of the plurality of target nucleic acids; (c) thermally cycling the mixture under reaction conditions appropriate to amplify the first and second members of the plurality of target nucleic acids with polymerase chain reaction (PCR), such that the first forward oligonucleotide primer and the second forward oligonucleotide primer are degraded; (d) thermally cycling the mixture under reaction conditions appropriate to amplify the template nucleic acid with PCR, such that a signal is generated if both the first and second members of the plurality of target nucleic acids are present in the mixture; and (e) detecting the presence or absence of the signal. In some embodiments, the method further comprises (f) correlating the presence of the signal to the presence of both the first and second members of the plurality of target nucleic acids in the sample. In some embodiments, the signal oligonucleotide probe comprises a signal tag. In some embodiments, in (d), the signal is generated by the signal tag. In some embodiments, the signal tag generates the signal upon degradation of the signal oligonucleotide probe. In some embodiments, the signal oligonucleotide probe has complementarity to a first region of the template nucleic acid. In some embodiments, the first forward oligonucleotide primer further comprises an additional region with complementarity to a second region of the template nucleic acid. In some embodiments, in (c), the additional region of the first forward oligonucleotide primer is released. In some embodiments, in (c), each thermal cycle is performed at an annealing temperature appropriate for annealing of the region of the first forward oligonucleotide primer to the first member of the plurality of target nucleic acids and inappropriate for annealing of the additional region of the first forward oligonucleotide primer to the first member of the plurality of target nucleic acids. In some embodiments, the additional region of the first forward oligonucleotide primer has between about 50% and about 100% complementarity to the template nucleic acid. In some embodiments, the additional region of the first forward oligonucleotide primer has between about 60% and about 100% complementarity to the template nucleic acid. In some embodiments, the additional region of the first forward oligonucleotide primer has between about 70% and about 100% complementarity to the template nucleic acid. In some embodiments, the additional region of the first forward oligonucleotide primer has between about 80% and about 100% complementarity to the template nucleic acid. In some embodiments, the additional region of the first forward oligonucleotide primer has between about 90% and about 100% complementarity to the template nucleic acid. In some embodiments, the additional region of the first forward oligonucleotide primer has between about 95% and about 100% complementarity to the template nucleic acid. In some embodiments, the additional region of the first forward oligonucleotide primer has between about 99% and about 100% complementarity to the template nucleic acid. In some embodiments, the additional region of the first forward oligonucleotide primer has about 100% complementarity to the template nucleic acid. In some embodiments, the additional region of the first forward oligonucleotide primer has length of between about 5 nucleotides and about 500 nucleotides. In some embodiments, the additional region of the first forward oligonucleotide primer has length of between about 5 nucleotides and about 300 nucleotides. In some embodiments, the additional region of the first forward oligonucleotide primer has length of between about 5 nucleotides and about 100 nucleotides. In some embodiments, the additional region of the first forward oligonucleotide primer has length of between about 5 nucleotides and about 50 nucleotides. In some embodiments, the additional region of the first forward oligonucleotide primer has length of between about 5 nucleotides and about 30 nucleotides. In some embodiments, the additional region of the first forward oligonucleotide primer has length of between about 5 nucleotides and about 20 nucleotides. In some embodiments, the additional region of the first forward oligonucleotide primer has length of between about 5 nucleotides and about 15 nucleotides. In some embodiments, the second forward oligonucleotide primer further comprises an additional region with complementarity to a third region of the template nucleic acid. In some embodiments, in (c), the additional region of the second forward oligonucleotide primer is released. In some embodiments, in (c), each thermal cycle is performed at an annealing temperature appropriate for annealing of the region of the second forward oligonucleotide primer to the second member of the plurality of target nucleic acids and inappropriate for annealing of the additional region of the second oligonucleotide primer to the second member of the plurality of target nucleic acids. In some embodiments, the first region of the template nucleic acid is 3' of the additional region of the template nucleic acid and 5' of the third region of the template nucleic acid. In some embodiments, the additional region of the second forward oligonucleotide primer has between about 50% and about 100% complementarity to the template nucleic acid. In some embodiments, the additional region of the second forward oligonucleotide primer has between about 60% and about 100% complementarity to the template nucleic acid. In some embodiments, the additional region of the second forward oligonucleotide primer has between about 70% and about 100% complementarity to the template nucleic acid. In some embodiments, the additional region of the second forward oligonucleotide primer has between about 80% and about 100% complementarity to the template nucleic acid. In some embodiments, the additional region of the second forward oligonucleotide primer has between about 90% and about 100% complementarity to the template nucleic acid. In some embodiments, the additional region of the second forward oligonucleotide primer has between about 95% and about 100% complementarity to the template nucleic acid. In some embodiments, the additional region of the second forward oligonucleotide primer has between about 99% and about 100% complementarity to the template nucleic acid. In some embodiments, the additional region of the second forward oligonucleotide primer has about 100% complementarity to the template nucleic acid. In some embodiments, the additional region of the first forward oligonucleotide primer is not identical to the additional region of the second forward oligonucleotide primer. In some embodiments, the additional region of the second forward oligonucleotide primer has length of between about 5 nucleotides and about 500 nucleotides. In some embodiments, the additional region of the second forward oligonucleotide primer has length of between about 5 nucleotides and about 300 nucleotides. In some embodiments, the additional region of the second forward oligonucleotide primer has length of between about 5 nucleotides and about 100 nucleotides. In some embodiments, the additional region of the second forward oligonucleotide primer has length of between about 5 nucleotides and about 50 nucleotides. In some embodiments, the additional region of the second forward oligonucleotide primer has length of between about 5 nucleotides and about 30 nucleotides. In some embodiments, the additional region of the second forward oligonucleotide primer has length of between about 5 nucleotides and about 20 nucleotides. In some embodiments, the additional region of the second forward oligonucleotide primer has length of between about 5 nucleotides and about 15 nucleotides. In some embodiments, the region of the first forward oligonucleotide primer has complementarity to a first region of the first member of the plurality of target nucleic acids. In some embodiments, the region of the first reverse oligonucleotide primer has complementarity to a second region of the first member of the plurality of target nucleic acids. In some embodiments, the region of the second forward oligonucleotide primer has complementarity to a first region of the second member of the plurality of target nucleic acids. In some embodiments, the region of the second reverse oligonucleotide primer has complementarity to a second region of the second member of the plurality of target nucleic acids. In some embodiments, mixture further comprises a nucleic acid enzyme. In some embodiments, in (c), the first and second oligonucleotide primers are degraded by the nucleic acid enzyme. In some embodiments, in (d), the signal oligonucleotide probe is degraded by the nucleic acid enzyme. In some embodiments, (c) and (d) are performed substantially simultaneously. In some embodiments, the signal oligonucleotide probe does not have complementarity to the any of the plurality of target nucleic acids. In some embodiments, the nucleic acid enzyme is a nucleic acid polymerase. In some embodiments, the nucleic acid enzyme has exonuclease activity. In some embodiments, the nucleic acid enzyme has endonuclease activity. In some embodiments, the first forward oligonucleotide primer and the second forward oligonucleotide primer are degraded by the endonuclease activity of the nucleic acid enzyme. In some embodiments, the signal is a fluorescent signal. In some embodiments, the signal tag comprises a fluorophore and a quencher. In some embodiments, the fluorophore is FAM, TET, HEX, JOE, Cy3, Cy5, or any combination thereof. In some embodiments, the method further comprises correlating the absence of the signal with the absence of both the first and second member of the plurality of target nucleic acids. In an aspect, the present disclosure provides a kit for detecting the presence or absence of a first and second member of a plurality of target nucleic acids in a sample, comprising: (a) a template nucleic acid; (b) a signal oligonucleotide probe with complementarity to a first region of the template nucleic acid; (c) a first oligonucleotide primer comprising: a first region with complementarity to a region of the first member of the plurality of target nucleic acids; and a second region with complementarity to a second region of the template nucleic acid; and (d) a second oligonucleotide primer comprising: a first region with complementarity to a region of the second member of the plurality of target nucleic acids; and a second region with complementarity to a third region of the template nucleic acid. In some embodiments, the kit comprises a plurality of the first and second oligonucleotide primers. In some embodiments, the signal oligonucleotide probe comprises a signal tag. In some embodiments, the signal oligonucleotide probe has complementarity to a first region of the template nucleic acid. In some embodiments, the kit further comprises (e) a third oligonucleotide primer comprising a region with complementarity to an additional region of the first member of the plurality of target nucleic acids; and (f) a fourth oligonucleotide primer comprising a region with complementarity to an additional region of the second member of the plurality of target nucleic acids. In some embodiments, the mixture comprises a plurality of the third and fourth oligonucleotide primers. In some embodiments, the kit further comprises a nucleic acid enzyme. In some embodiments, the nucleic acid enzyme is a nucleic acid polymerase. In some embodiments, the nucleic acid enzyme is an RNase. In some embodiments, the RNase is an RNase III. In some embodiments, the RNase III is Dicer. In some embodiments, the nucleic acid enzyme is an endonuclease. In some embodiments, the endonuclease is an endonuclease I. In some embodiments, the endonuclease I is T7 endonuclease I. In some embodiments, the signal oligonucleotide probe does not have complementarity to the any of the plurality of target nucleic acids. In some embodiments, the first region of the template nucleic acid is 3' of the second region of the template nucleic acid and 5' of the third region of the template nucleic acid. In some embodiments, the signal tag comprises a fluorophore and a quencher. In some embodiments, the fluorophore is FAM, TET, HEX, JOE, Cy3, Cy5, or any combination thereof. In some embodiments, the second region of the first oligonucleotide primer has between about 50% and about 100% complementarity to the template nucleic acid. In some embodiments, the second region of the first oligonucleotide primer has between about 60% and about 100% complementarity to the template nucleic acid. In some embodiments, the second region of the first oligonucleotide primer has between about 70% and about 100% complementarity to the template nucleic acid. In some embodiments, the second region of the first oligonucleotide primer has between about 80% and about 100% complementarity to the template nucleic acid. In some embodiments, the second region of the first oligonucleotide primer has between about 90% and about 100% complementarity to the template nucleic acid. In some embodiments, the second region of the first oligonucleotide primer has between about 95% and about 100% complementarity to the template nucleic acid. In some embodiments, the second region of the first oligonucleotide primer has between about 99% and about 100% complementarity to the template nucleic acid. In some embodiments, the second region of the first oligonucleotide primer has about 100% complementarity to the template nucleic acid. In some embodiments, the second region of the second oligonucleotide primer has between about 50% and about 100% complementarity to the template nucleic acid. In some embodiments, the second region of the second oligonucleotide primer has between about 60% and about 100% complementarity to the template nucleic acid. In some embodiments, the second region of the second oligonucleotide primer has between about 70% and about 100% complementarity to the template nucleic acid. In some embodiments, the second region of the second oligonucleotide primer has between about 80% and about 100% complementarity to the template nucleic acid. In some embodiments, the second region of the second oligonucleotide primer has between about 90% and about 100% complementarity to the template nucleic acid. In some embodiments, the second region of the second oligonucleotide primer has between about 95% and about 100% complementarity to the template nucleic acid. In some embodiments, the second region of the second oligonucleotide primer has between about 99% and about 100% complementarity to the template nucleic acid. In some embodiments, the second region of the second oligonucleotide primer has about 100% complementarity to the template nucleic acid. In some embodiments, the second region of the first oligonucleotide primer is not identical to the second region of the second oligonucleotide primer. In some embodiments, the second region of the first oligonucleotide primer has length of between about 5 nucleotides and about 500 nucleotides. In some embodiments, the second region of the first oligonucleotide primer has length of between about 5 nucleotides and about 300 nucleotides. In some embodiments, the second region of the first oligonucleotide primer has length of between about 5 nucleotides and about 100 nucleotides. In some embodiments, the second region of the first oligonucleotide primer has length of between about 5 nucleotides and about 50 nucleotides. In some embodiments, the second region of the first oligonucleotide primer has length of between about 5 nucleotides and about 30 nucleotides. In some embodiments, the second region of the first oligonucleotide primer has length of between about 5 nucleotides and about 20 nucleotides. In some embodiments, the second region of the first oligonucleotide primer has length of between about 5 nucleotides and about 15 nucleotides. In some embodiments, the second region of the second oligonucleotide primer has length of between about 5 nucleotides and about 500 nucleotides. In some embodiments, the second region of the second oligonucleotide primer has length of between about 5 nucleotides and about 300 nucleotides. In some embodiments, the second region of the second oligonucleotide primer has length of between about 5 nucleotides and about 100 nucleotides. In some embodiments, the second region of the second oligonucleotide primer has length of between about 5 nucleotides and about 50 nucleotides. In some embodiments, the second region of the second oligonucleotide primer has length of between about 5 nucleotides and about 30 nucleotides. In some embodiments, the second region of the second oligonucleotide primer has length of between about 5 nucleotides and about 20 nucleotides. In some embodiments, the second region of the second oligonucleotide primer has length of between about 5 nucleotides and about 15 nucleotides. In some embodiments, at least one of the oligonucleotide primers is lyophilized. In some embodiments, each of the oligonucleotide primers is lyophilized. In some embodiments, the signal oligonucleotide probe is lyophilized. In some embodiments, the template nucleic acid is lyophilized.

In an aspect, the present disclosure provides a method of detecting the presence or absence of at least one member of a plurality of target nucleic acids in a sample, the method comprising: (a) providing a sample comprising, or potentially comprising, at least one member of the plurality of target nucleic acids; (b) forming a mixture of the sample and (i) a signal oligonucleotide probe comprising a signal tag; (ii) a plurality of a first forward oligonucleotide primer comprising: a first region with complementarity to a first region of a first member of the plurality of target nucleic acids; and a second region with complementarity to the signal oligonucleotide probe; (iii) a plurality of a first reverse oligonucleotide primer comprising a region with complementarity to a second region of the first member of the plurality of target nucleic acids; (iv) a plurality of a second forward oligonucleotide primer comprising: a first region with complementarity to a first region of a second member of the plurality of target nucleic acids; and a second region with complementarity to the signal oligonucleotide probe; (v) a plurality of a second reverse oligonucleotide primer comprising a region with complementarity to a second region of the second member of the plurality of target nucleic acids; and (vi) a nucleic acid enzyme; (c) thermally cycling the mixture under reaction conditions appropriate to amplify each member of the plurality of target nucleic acids with polymerase chain reaction (PCR), such that the signal oligonucleotide probe is degraded by the nucleic acid enzyme such that a signal is generated if at least one member of the plurality of target nucleic acids is present in the mixture; (d) detecting the presence or absence of the signal; and (e) correlating the presence of the signal to the presence of at least one of the members of the plurality of target nucleic acids in the sample. In some embodiments, the mixture formed at (b) further comprises: a plurality of an nth forward oligonucleotide primer comprising: a first region with complementarity to a first region of an nth member of the plurality of target nucleic acids; and a second region with complementarity to the signal oligonucleotide probe; and a plurality of an nth reverse oligonucleotide primer comprising a region with complementarity to a second region of an nth member of the plurality of target nucleic acids; wherein n is an integer greater than 2. In some embodiments; the mixture formed at (b) further comprises: an xth signal oligonucleotide probe comprising an xth signal tag; a plurality of a yth forward oligonucleotide primer comprising: a first region with complementarity to a first region of a yth member of the plurality of target nucleic acids; and a second region with complementarity to the xth signal oligonucleotide probe; and a plurality of a yth reverse oligonucleotide primer comprising a region with complementarity to a second region of the yth member of the plurality of target nucleic acids; in (c), the xth signal oligonucleotide probe is degraded by the nucleic acid polymerase such that an xth signal is generated if at least one of the yth member of the plurality of target nucleic acids is present in the mixture; (e) further comprises correlating the presence of the xth signal to the presence of at least one of the yth members of the plurality target nucleic acids in the sample; and x is an integer greater than 1 and y is an integer greater than n. In some embodiments, the nucleic acid enzyme is a nucleic acid polymerase. In some embodiments, the nucleic acid enzyme has exonuclease activity. In some embodiments, the signal oligonucleotide probe is degraded by the exonuclease activity of the nucleic acid enzyme. In some embodiments, the nucleic acid enzyme has endonuclease activity. In some embodiments, the method further comprises correlating the absence of the signal with the absence of each member of the plurality of target nucleic acids. In some embodiments, the signal oligonucleotide probe does not have complementarity to any member of the plurality of target nucleic acids. In some embodiments, the first region of each of the plurality of first forward oligonucleotide primers is 5' relative to the second region of each of the plurality of first forward oligonucleotide primers. In some embodiments, the first region of each of the plurality of second forward oligonucleotide primers is 5' relative to the second region of each of the plurality of second forward oligonucleotide primers. In some embodiments, the first region of each of the plurality of nth forward oligonucleotide primers is 5' relative to the second region of each of the plurality of nth forward oligonucleotide primers. In some embodiments, the first region of each of the plurality of yth forward oligonucleotide primers is 5' relative to the second region of each of the plurality of yth forward oligonucleotide primers. In some embodiments, in (c), each thermal cycle is performed at an annealing temperature appropriate for annealing of the first region of each of the plurality of first forward oligonucleotide primers to the first member of the plurality of target nucleic acids and annealing of the second region of each of the plurality of first forward oligonucleotide primers to the signal oligonucleotide probe. In some embodiments, in (c), each thermal cycle is performed at an annealing temperature appropriate for annealing of the first region of each of the plurality of second forward oligonucleotide primers to the second member of the plurality of target nucleic acids and annealing of the second region of each of the plurality of second forward oligonucleotide primers to the signal oligonucleotide probe. In some embodiments, the second region of each of the plurality of first forward oligonucleotide primers has between about 50% and about 100% complementarity to the signal oligonucleotide probe. In some embodiments, the second region of each of the plurality of first forward oligonucleotide primers has between about 60% and about 100% complementarity to the signal oligonucleotide probe. In some embodiments, the second region of each of the plurality of first forward oligonucleotide primers has between about 70% and about 100% complementarity to the signal oligonucleotide probe. In some embodiments, the second region of each of the plurality of first forward oligonucleotide primers has between about 80% and about 100% complementarity to the signal oligonucleotide probe. In some embodiments, the second region of each of the plurality of first forward oligonucleotide primers has between about 90% and about 100% complementarity to the signal oligonucleotide probe. In some embodiments, the second region of each of the plurality of first forward oligonucleotide primers has between about 95% and about 100% complementarity to the signal oligonucleotide probe. In some embodiments, the second region of each of the plurality of first forward oligonucleotide primers has between about 99% and about 100% complementarity to the signal oligonucleotide probe. In some embodiments, the second region of each of the plurality of first forward oligonucleotide primers has about 100% complementarity to the signal oligonucleotide probe. In some embodiments, the second region of each of the plurality of second forward oligonucleotide primers has between about 50% and about 100% complementarity to the signal oligonucleotide probe. In some embodiments, the second region of each of the plurality of second forward oligonucleotide primers has between about 60% and about 100% complementarity to the signal oligonucleotide probe. In some embodiments, the second region of each of the plurality of second forward oligonucleotide primers has between about 70% and about 100% complementarity to the signal oligonucleotide probe. In some embodiments, the second region of each of the plurality of second forward oligonucleotide primers has between about 80% and about 100% complementarity to the signal oligonucleotide probe. In some embodiments, the second region of each of the plurality of second forward oligonucleotide primers has between about 90% and about 100% complementarity to the signal oligonucleotide probe. In some embodiments, the second region of each of the plurality of second forward oligonucleotide primers has between about 95% and about 100% complementarity to the signal oligonucleotide probe. In some embodiments, the second region of each of the plurality of second forward oligonucleotide primers has between about 99% and about 100% complementarity to the signal oligonucleotide probe. In some embodiments, the second region of each of the plurality of second forward oligonucleotide primers has about 100% complementarity to the signal oligonucleotide probe. In some embodiments, the second region of each of the plurality of first forward oligonucleotide primers has between about 50% and about 100% identity with the second region of each of the plurality of second forward oligonucleotide primers. In some embodiments, the second region of each of the plurality of first forward oligonucleotide primers has between about 50% and about 100% identity with the second region of each of the plurality of second forward oligonucleotide primers. In some embodiments, the second region of each of the plurality of first forward oligonucleotide primers has between about 60% and about 100% identity with the second region of each of the plurality of second forward oligonucleotide primers. In some embodiments, the second region of each of the plurality of first forward oligonucleotide primers has between about 70% and about 100% identity with the second region of each of the plurality of second forward oligonucleotide primers. In some embodiments, the second region of each of the plurality of first forward oligonucleotide primers has between about 80% and about 100% identity with the second region of each of the plurality of second forward oligonucleotide primers. In some embodiments, the second region of each of the plurality of first forward oligonucleotide primers has between about 90% and about 100% identity with the second region of each of the plurality of second forward oligonucleotide primers. In some embodiments, the second region of each of the plurality of first forward oligonucleotide primers has between about 95% and about 100% identity with the second region of each of the plurality of second forward oligonucleotide primers. In some embodiments, the second region of each of the plurality of first forward oligonucleotide primers has between about 99% and about 100% identity with the second region of each of the plurality of second forward oligonucleotide primers. In some embodiments, the second region of each of the plurality of first forward oligonucleotide primers has about 100% identity with the second region of each of the plurality of second forward oligonucleotide primers. In some embodiments, the second region of each of the plurality of first forward oligonucleotide primers has length of between about 5 nucleotides and about 500 nucleotides. In some embodiments, the second region of each of the plurality of first forward oligonucleotide primers has length of between about 5 nucleotides and about 300 nucleotides. In some embodiments, the second region of each of the plurality of first forward oligonucleotide primers has length of between about 5 nucleotides and about 100 nucleotides. In some embodiments, the second region of each of the plurality of first forward oligonucleotide primers has length of between about 5 nucleotides and about 50 nucleotides. In some embodiments, the second region of each of the plurality of first forward oligonucleotide primers has length of between about 5 nucleotides and about 30 nucleotides. In some embodiments, the second region of each of the plurality of first forward oligonucleotide primers has length of between about 5 nucleotides and about 20 nucleotides. In some embodiments, the second region of each of the plurality of first forward oligonucleotide primers has length of between about 5 nucleotides and about 15 nucleotides. In some embodiments, the second region of each of the plurality of second forward oligonucleotide primers has length of between about 5 nucleotides and about 500 nucleotides. In some embodiments, the second region of each of the plurality of second forward oligonucleotide primers has length of between about 5 nucleotides and about 300 nucleotides. In some embodiments, the second region of each of the plurality of second forward oligonucleotide primers has length of between about 5 nucleotides and about 100 nucleotides. In some embodiments, the second region of each of the plurality of second forward oligonucleotide primers has length of between about 5 nucleotides and about 50 nucleotides. In some embodiments, the second region of each of the plurality of second forward oligonucleotide primers has length of between about 5 nucleotides and about 30 nucleotides. In some embodiments, the second region of each of the plurality of second forward oligonucleotide primers has length of between about 5 nucleotides and about 20 nucleotides. In some embodiments, the second region of each of the plurality of second forward oligonucleotide primers has length of between about 5 nucleotides and about 15 nucleotides. In some embodiments, the nucleic acid enzyme is a deoxyribonucleic acid polymerase. In some embodiments, the deoxyribonucleic acid polymerase is a Taq polymerase. In some embodiments, the one or more target nucleic acids are deoxyribonucleic acids. In some embodiments, the one or more target nucleic acids are ribonucleic acids. In some embodiments, the signal is a fluorescent signal. In some embodiments, the signal tag comprises a fluorophore and a quencher. In some embodiments, the fluorophore is FAM, TET, HEX, JOE, Cy3, Cy5, or any combination thereof. In some embodiments, the signal tags are different. In some embodiments, the signal tags are the same. In some embodiments, the signals are different signal types. In some embodiments, the signals are different intensities of the same signal type.

In an aspect, the present disclosure provides a kit for detecting the presence or absence of at least one member of a plurality of target nucleic acids in a sample, comprising: (a) a signal oligonucleotide probe comprising a signal tag; (b) a plurality of a first forward oligonucleotide primer comprising: a first region with complementarity to a first region of a first member of the plurality of target nucleic acids; and a second region with complementarity to the signal oligonucleotide probe; (c) a plurality of a first reverse oligonucleotide primer comprising a region with complementarity to a second region of the first member of the plurality of target nucleic acids; (d) a plurality of a second forward oligonucleotide primer comprising: a first region with complementarity to a first region of a second member of the plurality of target nucleic acids; and a second region with complementarity to the signal oligonucleotide probe; (e) a plurality of a second reverse oligonucleotide primer comprising a region with complementarity to a second region of the second member of the plurality of target nucleic acids. In some embodiments, the kit further comprises: a plurality of an nth forward oligonucleotide primer comprising: a first region with complementarity to a first region of an nth member of the plurality of target nucleic acids; and a second region with complementarity to the signal oligonucleotide probe; and a plurality of an nth reverse oligonucleotide primer comprising a region with complementarity to a second region of an nth member of the plurality of target nucleic acids; wherein n is an integer greater than 2. In some embodiments, the kit further comprises: an xth signal oligonucleotide probe comprising an xth signal tag; a plurality of a yth forward oligonucleotide primer comprising: a first region with complementarity to a first region of a yth member of the plurality of target nucleic acids; and a second region with complementarity to the xth signal oligonucleotide probe; and a plurality of a yth reverse oligonucleotide primer comprising a region with complementarity to a second region of the yth member of the plurality of target nucleic acids; wherein x is an integer greater than 1 and y is an integer greater than n. In some embodiments, the signal tags are different. In some embodiments, the signal tags are the same. In some embodiments, the signal oligonucleotide probes are present in different concentrations. In some embodiments, at least one of the oligonucleotide primers is lyophilized. In some embodiments, each of the oligonucleotide primers is lyophilized. In some embodiments, the signal oligonucleotide probe is lyophilized. In some embodiments, the signal tag comprises a fluorophore and a quencher. In some embodiments, the fluorophore is FAM, TET, HEX, JOE, Cy3, Cy5, or any combination thereof. In some embodiments, the second region of each of the plurality of first forward oligonucleotide primers has between about 50% and about 100% complementarity to the signal oligonucleotide probe. In some embodiments, the second region of each of the plurality of first forward oligonucleotide primers has between about 60% and about 100% complementarity to the signal oligonucleotide probe. In some embodiments, the second region of each of the plurality of first forward oligonucleotide primers has between about 70% and about 100% complementarity to the signal oligonucleotide probe. In some embodiments, the second region of each of the plurality of first forward oligonucleotide primers has between about 80% and about 100% complementarity to the signal oligonucleotide probe. In some embodiments, the second region of each of the plurality of first forward oligonucleotide primers has between about 90% and about 100% complementarity to the signal oligonucleotide probe. In some embodiments, the second region of each of the plurality of first forward oligonucleotide primers has between about 95% and about 100% complementarity to the signal oligonucleotide probe. In some embodiments, the second region of each of the plurality of first forward oligonucleotide primers has between about 99% and about 100% complementarity to the signal oligonucleotide probe. In some embodiments, the second region of each of the plurality of first forward oligonucleotide primers has about 100% complementarity to the signal oligonucleotide probe. In some embodiments, the second region of each of the plurality of second forward oligonucleotide primers has between about 50% and about 100% complementarity to the signal oligonucleotide probe. In some embodiments, the second region of each of the plurality of second forward oligonucleotide primers has between about 60% and about 100% complementarity to the signal oligonucleotide probe. In some embodiments, the second region of each of the plurality of second forward oligonucleotide primers has between about 70% and about 100% complementarity to the signal oligonucleotide probe. In some embodiments, the second region of each of the plurality of second forward oligonucleotide primers has between about 80% and about 100% complementarity to the signal oligonucleotide probe. In some embodiments, the second region of each of the plurality of second forward oligonucleotide primers has between about 90% and about 100% complementarity to the signal oligonucleotide probe. In some embodiments, the second region of each of the plurality of second forward oligonucleotide primers has between about 95% and about 100% complementarity to the signal oligonucleotide probe. In some embodiments, the second region of each of the plurality of second forward oligonucleotide primers has between about 99% and about 100% complementarity to the signal oligonucleotide probe. In some embodiments, the second region of each of the plurality of second forward oligonucleotide primers has about 100% complementarity to the signal oligonucleotide probe.. In some embodiments, the second region of each of the plurality of first forward oligonucleotide primers has between about 50% and about 100% identity with the second region of each of the plurality of second forward oligonucleotide primers. In some embodiments, the second region of each of the plurality of first forward oligonucleotide primers has between about 50% identity with the second region of each of the plurality of second forward oligonucleotide primers. In some embodiments, the second region of each of the plurality of first forward oligonucleotide primers has between about 60% and about 100% identity with the second region of each of the plurality of second forward oligonucleotide primers. In some embodiments, the second region of each of the plurality of first forward oligonucleotide primers has between about 70% and about 100% identity with the second region of each of the plurality of second forward oligonucleotide primers. In some embodiments, the second region of each of the plurality of first forward oligonucleotide primers has between about 80% and about 100% identity with the second region of each of the plurality of second forward oligonucleotide primers. In some embodiments, the second region of each of the plurality of first forward oligonucleotide primers has between about 90% and about 100% identity with the second region of each of the plurality of second forward oligonucleotide primers. In some embodiments, the second region of each of the plurality of first forward oligonucleotide primers has between about 95% and about 100% identity with the second region of each of the plurality of second forward oligonucleotide primers. In some embodiments, the second region of each of the plurality of first forward oligonucleotide primers has between about 99% and about 100% identity with the second region of each of the plurality of second forward oligonucleotide primers. In some embodiments, the second region of each of the plurality of first forward oligonucleotide primers has about 100% identity with the second region of each of the plurality of second forward oligonucleotide primers. In some embodiments, the second region of each of the plurality of first forward oligonucleotide primers has length of between about 5 nucleotides and about 500 nucleotides. In some embodiments, the second region of each of the plurality of first forward oligonucleotide primers has length of between about 5 nucleotides and about 300 nucleotides. In some embodiments, the second region of each of the plurality of first forward oligonucleotide primers has length of between about 5 nucleotides and about 100 nucleotides. In some embodiments, the second region of each of the plurality of first forward oligonucleotide primers has length of between about 5 nucleotides and about 50 nucleotides. In some embodiments, the second region of each of the plurality of first forward oligonucleotide primers has length of between about 5 nucleotides and about 30 nucleotides. In some embodiments, the second region of each of the plurality of first forward oligonucleotide primers has length of between about 5 nucleotides and about 20 nucleotides. In some embodiments, the second region of each of the plurality of first forward oligonucleotide primers has length of between about 5 nucleotides and about 15 nucleotides. In some embodiments, the second region of each of the plurality of second forward oligonucleotide primers has length of between about 5 nucleotides and about 500 nucleotides. In some embodiments, the second region of each of the plurality of second forward oligonucleotide primers has length of between about 5 nucleotides and about 300 nucleotides. In some embodiments, the second region of each of the plurality of second forward oligonucleotide primers has length of between about 5 nucleotides and about 100 nucleotides. In some embodiments, the second region of each of the plurality of second forward oligonucleotide primers has length of between about 5 nucleotides and about 50 nucleotides. In some embodiments, the second region of each of the plurality of second forward oligonucleotide primers has length of between about 5 nucleotides and about 30 nucleotides. In some embodiments, the second region of each of the plurality of second forward oligonucleotide primers has length of between about 5 nucleotides and about 20 nucleotides. In some embodiments, the second region of each of the plurality of second forward oligonucleotide primers has length of between about 5 nucleotides and about 15 nucleotides.

In an aspect, the present disclosure provides a method of detecting the presence or absence of a first and second member of a plurality of target nucleic acids in a sample, the method comprising: (a) providing a sample comprising, or potentially comprising, the first and second members of the plurality of target nucleic acids (b) forming a mixture comprising the sample and (i) a template nucleic acid; (ii) a signal oligonucleotide probe with complementarity to a first region of the template nucleic acid, the signal oligonucleotide probe comprising a signal tag; (iii) a plurality of a first oligonucleotide primer comprising: a first region with complementarity to a region of the first member of the plurality of target nucleic acids; and a second region with complementarity to a second region of the template nucleic acid; (iv) a plurality of a second forward oligonucleotide primer comprising: a first region with complementarity to a region of the second member of the plurality of target nucleic acids; and a second region with complementarity to a third region of the template nucleic acid; and (v) one or more nucleic acid enzymes; (c) subjecting the mixture to conditions sufficient to anneal the first oligonucleotide primer to the first member of the plurality of target nucleic acids and the second oligonucleotide primer to the second member of the plurality of target nucleic acids, such that the first and second oligonucleotide primers are degraded by the nucleic acid enzyme, releasing the second region of the first oligonucleotide primer and the second region of the second oligonucleotide primer; (d) thermally cycling the mixture under reaction conditions appropriate to amplify the template nucleic acid with polymerase chain reaction (PCR), such that the signal oligonucleotide probe is degraded such that a signal is generated if both the first and second members of the plurality of target nucleic acids are present in the mixture; (e) detecting the presence or absence of the signal; and (f) correlating the presence of the signal to the presence of both the first and second members of the plurality of target nucleic acids in the sample. In some embodiments, the mixture in (b) further comprises (vi) a plurality of a third oligonucleotide primer comprising a region with complementarity to a second region of the first member of the plurality of target nucleic acids; and (vii) a plurality of a fourth oligonucleotide primer comprising a region with complementarity to a second region of the second member of the plurality of target nucleic acids. In some embodiments, in (c), the conditions are sufficient to denature the plurality of target nucleic acids. In some embodiments, the method further comprises in (c), thermally cycling the mixture under reaction conditions appropriate to amplify the first and second members of the plurality of target nucleic acids with polymerase chain reaction. In some embodiments, the nucleic acid enzyme is a nucleic acid polymerase. In some embodiments, the nucleic acid enzyme has exonuclease activity. In some embodiments, the signal oligonucleotide probe is degraded by the exonuclease activity of the nucleic acid enzyme. In some embodiments, the nucleic acid enzyme has endonuclease activity. In some embodiments, the first oligonucleotide primer and the second oligonucleotide primer are degraded by the endonuclease activity of the nucleic acid enzyme. In some embodiments, the plurality of target nucleic acids comprises a plurality of deoxyribonucleic acids. In some embodiments, the plurality of target nucleic acids comprises a plurality of ribonucleic acids. In some embodiments, the plurality of ribonucleic acids comprises a plurality of messenger ribonucleic acids. In some embodiments, the template nucleic acid is a deoxyribonucleic acid. In some embodiments, the template nucleic acid is a ribonucleic acid. In some embodiments, the nucleic acid enzyme has RNase activity. In some embodiments, the first oligonucleotide primer and the second oligonucleotide primer are degraded by RNase activity of the nucleic acid enzyme. In some embodiments, the nucleic acid enzyme is an RNase H. In some embodiments, the nucleic acid enzyme is an RNase III. In some embodiments, the RNase III is Dicer. In some embodiments, the nucleic acid enzyme is an endonuclease I. In some embodiments, the endonuclease I is T7 endonuclease I. In some embodiments, the nucleic acid enzyme is capable of degrading a nucleic acid comprising a non-natural nucleotide. In some embodiments, the enzyme is an endonuclease V. In some embodiments, the endonuclease V is E. coli endonuclease V. In some embodiments, the first region of each of the plurality of first oligonucleotide primers comprises one or more ribonucleotide bases. In some embodiments, the second region of each of the plurality of the first oligonucleotide primers comprises deoxyribonucleic acid. In some embodiments, the first region of each of the plurality of second oligonucleotide primers comprises one or more ribonucleotide bases. In some embodiments, the second region of each of the plurality of the second oligonucleotide primers comprises deoxyribonucleic acid. In some embodiments, the plurality of first oligonucleotide primers further comprises at least one non-natural nucleotide. In some embodiments, the non-natural nucleotide is deoxyinosine. In some embodiments, the plurality of second oligonucleotide primers further comprises at least one non-natural nucleotide. In some embodiments, the non-natural nucleotide is deoxyinosine. In some embodiments, in (c), the conditions are insufficient for annealing of the second region of the first oligonucleotide primer to the first member of the plurality of target nucleic acids. In some embodiments, in (c), the conditions are insufficient for annealing of the second region of the second oligonucleotide primer to the second member of the plurality of target nucleic acids. In some embodiments, the signal oligonucleotide probe does not have complementarity to the any of the plurality of target nucleic acids. In some embodiments, the first region of the template nucleic acid is 3' of the second region of the template nucleic acid and 5' of the third region of the template nucleic acid. In some embodiments, the signal tag comprises a fluorophore and a quencher. In some embodiments, the fluorophore is FAM, TET, HEX, JOE, Cy3, Cy5, or any combination thereof. In some embodiments, the second region of each of the plurality of first oligonucleotide primers has between about 50% and about 100% complementarity to the template nucleic acid. In some embodiments, the second region of each of the plurality of first oligonucleotide primers has between about 60% and about 100% complementarity to the template nucleic acid. In some embodiments, the second region of each of the plurality of first oligonucleotide primers has between about 70% and about 100% complementarity to the template nucleic acid. In some embodiments, the second region of each of the plurality of first oligonucleotide primers has between about 80% and about 100% complementarity to the template nucleic acid. In some embodiments, the second region of each of the plurality of first oligonucleotide primers has between about 90% and about 100% complementarity to the template nucleic acid. In some embodiments, the second region of each of the plurality of first oligonucleotide primers has between about 95% and about 100% complementarity to the template nucleic acid. In some embodiments, the second region of each of the plurality of first oligonucleotide primers has between about 99% and about 100% complementarity to the template nucleic acid. In some embodiments, the second region of each of the plurality of first oligonucleotide primers has about 100% complementarity to the template nucleic acid. In some embodiments, the second region of each of the plurality of second oligonucleotide primers has between about 50% and about 100% complementarity to the template nucleic acid. In some embodiments, the second region of each of the plurality of second oligonucleotide primers has between about 60% and about 100% complementarity to the template nucleic acid. In some embodiments, the second region of each of the plurality of second oligonucleotide primers has between about 70% and about 100% complementarity to the template nucleic acid. In some embodiments, the second region of each of the plurality of second oligonucleotide primers has between about 80% and about 100% complementarity to the template nucleic acid. In some embodiments, the second region of each of the plurality of second oligonucleotide primers has between about 90% and about 100% complementarity to the template nucleic acid. In some embodiments, the second region of each of the plurality of second oligonucleotide primers has between about 95% and about 100% complementarity to the template nucleic acid. In some embodiments, the second region of each of the plurality of second oligonucleotide primers has between about 99% and about 100% complementarity to the template nucleic acid. In some embodiments, the second region of each of the plurality of second oligonucleotide primers has about 100% complementarity to the template nucleic acid. In some embodiments, the second region of each of the plurality of first oligonucleotide primers is not identical to the second region of each of the plurality of second oligonucleotide primers. In some embodiments, the second region of each of the plurality of first oligonucleotide primers has length of between about 5 nucleotides and about 500 nucleotides. In some embodiments, the second region of each of the plurality of first oligonucleotide primers has length of between about 5 nucleotides and about 300 nucleotides. In some embodiments, the second region of each of the plurality of first oligonucleotide primers has length of between about 5 nucleotides and about 100 nucleotides. In some embodiments, the second region of each of the plurality of first oligonucleotide primers has length of between about 5 nucleotides and about 50 nucleotides. In some embodiments, the second region of each of the plurality of first oligonucleotide primers has length of between about 5 nucleotides and about 30 nucleotides. In some embodiments, the second region of each of the plurality of first oligonucleotide primers has length of between about 5 nucleotides and about 20 nucleotides. In some embodiments, the second region of each of the plurality of first oligonucleotide primers has length of between about 5 nucleotides and about 15 nucleotides. In some embodiments, the second region of each of the plurality of second oligonucleotide primers has length of between about 5 nucleotides and about 500 nucleotides. In some embodiments, the second region of each of the plurality of second oligonucleotide primers has length of between about 5 nucleotides and about 300 nucleotides. In some embodiments, the second region of each of the plurality of second oligonucleotide primers has length of between about 5 nucleotides and about 100 nucleotides. In some embodiments, the second region of each of the plurality of second oligonucleotide primers has length of between about 5 nucleotides and about 50 nucleotides. In some embodiments, the second region of each of the plurality of second oligonucleotide primers has length of between about 5 nucleotides and about 30 nucleotides. In some embodiments, the second region of each of the plurality of second oligonucleotide primers has length of between about 5 nucleotides and about 20 nucleotides. In some embodiments, the second region of each of the plurality of second oligonucleotide primers has length of between about 5 nucleotides and about 15 nucleotides. In some embodiments, the method further comprises correlating the absence of the signal with the absence of both the first and second member of the plurality of target nucleic acids.

In an aspect, the present disclosure provides a method of detecting the presence or absence of a first and second member of a plurality of target nucleic acids in a sample, the method comprising: (a) providing a sample comprising, or potentially comprising, the first and second members of the plurality of target nucleic acids (b) forming a mixture comprising the sample and (i) a template nucleic acid; (ii) a signal oligonucleotide probe with complementarity to a first region of the template nucleic acid, the signal oligonucleotide probe comprising a signal tag; (iii) a plurality of a first forward oligonucleotide primer comprising: a first region with complementarity to a first region of the first member of the plurality of target nucleic acids; and a second region with complementarity to a second region of the template nucleic acid; (iv) a plurality of a first reverse oligonucleotide primer comprising a region with complementarity to a second region of the first member of the plurality of target nucleic acids; (v) a plurality of a second forward oligonucleotide primer comprising: a first region with complementarity to a first region of the second member of the plurality of target nucleic acids; and a second region with complementarity to a third region of the template nucleic acid; (vi) a plurality of a second reverse oligonucleotide primer comprising a region with complementarity to a second region of the second member of the plurality of target nucleic acids; and (vii) a nucleic acid enzyme; (c) thermally cycling the mixture under reaction conditions appropriate to amplify the first and second members of the plurality of target nucleic acids with polymerase chain reaction (PCR), such that the first forward oligonucleotide primer and the second forward oligonucleotide primer are degraded by the nucleic acid enzyme, releasing the second region with complementarity to the second region of the template nucleic acid and the second region with complementarity to the third region of the template nucleic acid; (d) thermally cycling the mixture under reaction conditions appropriate to amplify the template nucleic acid with PCR, such that the signal oligonucleotide probe is degraded such that a signal is generated if both the first and second members of the plurality of target nucleic acids are present in the mixture; (e) detecting the presence or absence of the signal; and (f) correlating the presence of the signal to the presence of both the first and second members of the plurality of target nucleic acids in the sample. In some embodiments, in (c), each thermal cycle is performed at an annealing temperature appropriate for annealing of the first region of the first oligonucleotide primer to the first member of the plurality of target nucleic acids and inappropriate for annealing of the second region of the first oligonucleotide primer to the first member of the plurality of target nucleic acids. In some embodiments, in (c), each thermal cycle is performed at an annealing temperature appropriate for annealing of the first region of the second oligonucleotide primer to the second member of the plurality of target nucleic acids and inappropriate for annealing of the second region of the second oligonucleotide primer to the second member of the plurality of target nucleic acids. In some embodiments, (c) and (d) are performed substantially simultaneously. In some embodiments, the signal oligonucleotide probe does not have complementarity to the any of the plurality of target nucleic acids. In some embodiments, the first region of the template nucleic acid is 3' of the second region of the template nucleic acid and 5' of the third region of the template nucleic acid. In some embodiments, the nucleic acid enzyme is a nucleic acid polymerase. In some embodiments, the nucleic acid enzyme has exonuclease activity. In some embodiments, the nucleic acid enzyme has endonuclease activity. In some embodiments, the first forward oligonucleotide primer and the second forward oligonucleotide primer are degraded by the endonuclease activity of the nucleic acid enzyme. In some embodiments, the signal is a fluorescent signal. In some embodiments, the signal tag comprises a fluorophore and a quencher. In some embodiments, the fluorophore is FAM, TET, HEX, JOE, Cy3, Cy5, or any combination thereof. In some embodiments, the second region of each of the plurality of first forward oligonucleotide primers has between about 50% and about 100% complementarity to the template nucleic acid. In some embodiments, the second region of each of the plurality of first forward oligonucleotide primers has between about 60% and about 100% complementarity to the template nucleic acid. In some embodiments, the second region of each of the plurality of first forward oligonucleotide primers has between about 70% and about 100% complementarity to the template nucleic acid. In some embodiments, the second region of each of the plurality of first forward oligonucleotide primers has between about 80% and about 100% complementarity to the template nucleic acid. In some embodiments, the second region of each of the plurality of first forward oligonucleotide primers has between about 90% and about 100% complementarity to the template nucleic acid. In some embodiments, the second region of each of the plurality of first forward oligonucleotide primers has between about 95% and about 100% complementarity to the template nucleic acid. In some embodiments, the second region of each of the plurality of first forward oligonucleotide primers has between about 99% and about 100% complementarity to the template nucleic acid. In some embodiments, the second region of each of the plurality of first forward oligonucleotide primers has about 100% complementarity to the template nucleic acid. In some embodiments, the second region of each of the plurality of second forward oligonucleotide primers has between about 50% and about 100% complementarity to the template nucleic acid. In some embodiments, the second region of each of the plurality of second forward oligonucleotide primers has between about 60% and about 100% complementarity to the template nucleic acid. In some embodiments, the second region of each of the plurality of second forward oligonucleotide primers has between about 70% and about 100% complementarity to the template nucleic acid. In some embodiments, the second region of each of the plurality of second forward oligonucleotide primers has between about 80% and about 100% complementarity to the template nucleic acid. In some embodiments, the second region of each of the plurality of second forward oligonucleotide primers has between about 90% and about 100% complementarity to the template nucleic acid. In some embodiments, the second region of each of the plurality of second forward oligonucleotide primers has between about 95% and about 100% complementarity to the template nucleic acid. In some embodiments, the second region of each of the plurality of second forward oligonucleotide primers has between about 99% and about 100% complementarity to the template nucleic acid. In some embodiments, the second region of each of the plurality of second forward oligonucleotide primers has about 100% complementarity to the template nucleic acid. In some embodiments, the second region of each of the plurality of first forward oligonucleotide primers is not identical to the second region of each of the plurality of second forward oligonucleotide primers. In some embodiments, the second region of each of the plurality of first forward oligonucleotide primers has length of between about 5 nucleotides and about 500 nucleotides. In some embodiments, the second region of each of the plurality of first forward oligonucleotide primers has length of between about 5 nucleotides and about 300 nucleotides. In some embodiments, the second region of each of the plurality of first forward oligonucleotide primers length of between about 5 nucleotides and about 100 nucleotides. In some embodiments, the second region of each of the plurality of first forward oligonucleotide primers has length of between about 5 nucleotides and about 50 nucleotides. In some embodiments, the second region of each of the plurality of first forward oligonucleotide primers has length of between about 5 nucleotides and about 30 nucleotides. In some embodiments, the second region of each of the plurality of first forward oligonucleotide primers has length of between about 5 nucleotides and about 20 nucleotides. In some embodiments, the second region of each of the plurality of first forward oligonucleotide primers has length of between about 5 nucleotides and about 15 nucleotides. In some embodiments, the second region of each of the plurality of second forward oligonucleotide primers has length of between about 5 nucleotides and about 500 nucleotides. In some embodiments, the second region of each of the plurality of second forward oligonucleotide primers has length of between about 5 nucleotides and about 300 nucleotides. In some embodiments, the second region of each of the plurality of second forward oligonucleotide primers has length of between about 5 nucleotides and about 100 nucleotides. In some embodiments, the second region of each of the plurality of second forward oligonucleotide primers has length of between about 5 nucleotides and about 50 nucleotides. In some embodiments, the second region of each of the plurality of second forward oligonucleotide primers has length of between about 5 nucleotides and about 30 nucleotides. In some embodiments, the second region of each of the plurality of second forward oligonucleotide primers has length of between about 5 nucleotides and about 20 nucleotides. In some embodiments, the second region of each of the plurality of second forward oligonucleotide primers has length of between about 5 nucleotides and about 15 nucleotides. In some embodiments, the method further comprises correlating the absence of the signal with the absence of both the first and second members of the plurality of target nucleic acids.

In an aspect, the present disclosure provides a kit for detecting the presence or absence of a first and second member of a plurality of target nucleic acids in a sample, comprising: (a) a template nucleic acid; (b) a signal oligonucleotide probe with complementarity to a first region of the template nucleic acid, the signal oligonucleotide probe comprising a signal tag; (c) a plurality of a first oligonucleotide primer comprising: a first region with complementarity to a region of the first member of the plurality of target nucleic acids; and a second region with complementarity to a second region of the template nucleic acid; and (d) a plurality of a second oligonucleotide primer comprising: a first region with complementarity to a region of the second member of the plurality of target nucleic acids; and a second region with complementarity to a third region of the template nucleic acid. In some embodiments, the kit further comprises (e) a plurality of a third oligonucleotide primer comprising a region with complementarity to a second region of the first member of the plurality of target nucleic acids; and (f) a plurality of a fourth oligonucleotide primer comprising a region with complementarity to a second region of the second member of the plurality of target nucleic acids. In some embodiments, the kit further comprises a nucleic acid enzyme. In some embodiments, the nucleic acid enzyme is a nucleic acid polymerase. In some embodiments, the nucleic acid enzyme is an RNase. In some embodiments, the RNase is an RNase III. In some embodiments, the RNase III is Dicer. In some embodiments, the nucleic acid enzyme is an endonuclease. In some embodiments, the endonuclease is an endonuclease I. In some embodiments, the endonuclease I is T7 endonuclease I. In some embodiments, the signal oligonucleotide probe does not have complementarity to the any of the plurality of target nucleic acids. In some embodiments, the first region of the template nucleic acid is 3' of the second region of the template nucleic acid and 5' of the third region of the template nucleic acid. In some embodiments, the signal tag comprises a fluorophore and a quencher. In some embodiments, the fluorophore is FAM, TET, HEX, JOE, Cy3, Cy5, or any combination thereof. In some embodiments, the second region of each of the plurality of first oligonucleotide primers has between about 50% and about 100% complementarity to the template nucleic acid. In some embodiments, the second region of each of the plurality of first oligonucleotide primers has between about 60% and about 100% complementarity to the template nucleic acid. In some embodiments, the second region of each of the plurality of first oligonucleotide primers has between about 70% and about 100% complementarity to the template nucleic acid. In some embodiments, the second region of each of the plurality of first oligonucleotide primers has between about 80% and about 100% complementarity to the template nucleic acid. In some embodiments, the second region of each of the plurality of first oligonucleotide primers has between about 90% and about 100% complementarity to the template nucleic acid. In some embodiments, the second region of each of the plurality of first oligonucleotide primers has between about 95% and about 100% complementarity to the template nucleic acid. In some embodiments, the second region of each of the plurality of first oligonucleotide primers has between about 99% and about 100% complementarity to the template nucleic acid. In some embodiments, the second region of each of the plurality of first oligonucleotide primers has about 100% complementarity to the template nucleic acid. In some embodiments, the second region of each of the plurality of second oligonucleotide primers has between about 50% and about 100% complementarity to the template nucleic acid. In some embodiments, the second region of each of the plurality of second oligonucleotide primers has between about 60% and about 100% complementarity to the template nucleic acid. In some embodiments, the second region of each of the plurality of second oligonucleotide primers has between about 70% and about 100% complementarity to the template nucleic acid. In some embodiments, the second region of each of the plurality of second oligonucleotide primers has between about 80% and about 100% complementarity to the template nucleic acid. In some embodiments, the second region of each of the plurality of second oligonucleotide primers has between about 90% and about 100% complementarity to the template nucleic acid. In some embodiments, the second region of each of the plurality of second oligonucleotide primers has between about 95% and about 100% complementarity to the template nucleic acid. In some embodiments, the second region of each of the plurality of second oligonucleotide primers has between about 99% and about 100% complementarity to the template nucleic acid. In some embodiments, the second region of each of the plurality of second oligonucleotide primers has about 100% complementarity to the template nucleic acid. In some embodiments, the second region of each of the plurality of first oligonucleotide primers is not identical to the second region of each of the plurality of second oligonucleotide primers. In some embodiments, the second region of each of the plurality of first oligonucleotide primers has length of between about 5 nucleotides and about 500 nucleotides. In some embodiments, the second region of each of the plurality of first oligonucleotide primers has length of between about 5 nucleotides and about 300 nucleotides. In some embodiments, the second region of each of the plurality of first oligonucleotide primers has length of between about 5 nucleotides and about 100 nucleotides. In some embodiments, the second region of each of the plurality of first oligonucleotide primers has length of between about 5 nucleotides and about 50 nucleotides. In some embodiments, the second region of each of the plurality of first oligonucleotide primers has length of between about 5 nucleotides and about 30 nucleotides. In some embodiments, the second region of each of the plurality of first oligonucleotide primers has length of between about 5 nucleotides and about 20 nucleotides. In some embodiments, the second region of each of the plurality of first oligonucleotide primers has length of between about 5 nucleotides and about 15 nucleotides. In some embodiments, the second region of each of the plurality of second oligonucleotide primers has length of between about 5 nucleotides and about 500 nucleotides. In some embodiments, the second region of each of the plurality of second oligonucleotide primers has length of between about 5 nucleotides and about 300 nucleotides. In some embodiments, the second region of each of the plurality of second oligonucleotide primers has length of between about 5 nucleotides and about 100 nucleotides. In some embodiments, the second region of each of the plurality of second oligonucleotide primers has length of between about 5 nucleotides and about 50 nucleotides. In some embodiments, the second region of each of the plurality of second oligonucleotide primers has length of between about 5 nucleotides and about 30 nucleotides. In some embodiments, the second region of each of the plurality of second oligonucleotide primers has length of between about 5 nucleotides and about 20 nucleotides. In some embodiments, the second region of each of the plurality of second oligonucleotide primers has length of between about 5 nucleotides and about 15 nucleotides. In some embodiments, at least one of the oligonucleotide primers is lyophilized. In some embodiments, each of the oligonucleotide primers is lyophilized. In some embodiments, the signal oligonucleotide probe is lyophilized. In some embodiments, the template nucleic acid is lyophilized.

### BRIEF DESCRIPTION OF THE DRAWINGS

FIG. 1 shows an example method for generating a signal for detecting the presence of one or more of multiple members of a plurality of target nucleic acids.
FIG. 2 shows an example method for generating a signal for detecting the presence of one member of a plurality of target nucleic acids.
FIG. 3 shows an example method for generating a signal for detecting the presence of a first and second member of a plurality of target nucleic acids.
FIG. 4 shows another example method for generating a signal for detecting the presence of a first and second member of a plurality of target nucleic acids.
FIG. 5 illustrates an example analysis of the present disclosure.
FIG. 6 shows an example method for generating a signal for detecting the presence of one or more of multiple target nucleic acids using a universal primer.
FIG. 7 shows results from an example quantitative polymerase chain reaction experiment measuring the presence or absence of at least one of two targets.
FIG. 8 shows peak fluorescence intensity results from the example experiment referred to in FIG. 7.

### DETAILED DESCRIPTION

Polymerase Chain Reaction (PCR) is a method of exponential amplification of specific target nucleic acid in a reaction mix with a nucleic acid polymerase and primers. Primers are short single stranded oligonucleotides which are complementary to the 3' sequences of the positive and negative strand of the target sequence. The reaction mix is cycled in repeated heating and cooling steps. The heating cycle denatures or splits a double stranded nucleic acid target into single stranded templates. In the cooling cycle, the primers bind to complementary sequence on the template. After the template is primed the nucleic acid polymerase creates a copy of the original template. Repeated cycling exponentially amplifies the target 2 fold with each cycle leading to approximately a billion-fold increase of the target sequence in 30 cycles (Saiki et al 1988).

Real-Time PCR is a process of monitoring a PCR reaction by recording the fluorescence generated either by an intercalating dye such as SYBR Green or a target-specific reporter probe at each cycle. This is generally performed on a Real-Time PCR instrument that executes thermal cycling of the sample to complete the PCR cycles and at a specified point in each cycle measures the fluorescence of the sample in each channel through a series of excitation/emission filter sets.

Frequently, the target specific reporter probe is a short oligonucleotide complementary to one strand of the amplified target. The probe lacks a 3' hydroxyl and therefore is not extendable by the DNA polymerase. TaqMan (ThermoFisher Scientific) chemistry is a common reporter probe method used for multiplex Real-Time PCR (Holland et al. 1991). The TaqMan oligonucleotide probe is covalently modified with a fluorophore and a quenching tag (i.e., quencher). In this configuration the fluorescence generated by the fluorophore is quenched and is not detected by the real time PCR instrument. When the target of interest is present, the probe oligonucleotide base pairs with the amplified target. While bound, it is digested by the 5'->3' exonuclease activity of the Taq polymerase thereby physically separating the fluorophore from the quencher and liberating signal for detection by the real time PCR instrument.

Current methods that use a unique probe for each target of interest limit the number of targets that can be measured in a single reaction. While current multiplex technology allows for measurement of multiple targets using multiple unique probes, this technology is costly and is limited by the number of available fluorescent channels. There remains a need for methods which allow for measurement of any subset of a number of targets in a single sample. Disclosed herein are methods and compositions for detecting the presence or absence of one or more members of a plurality of target nucleic acids in a sample.

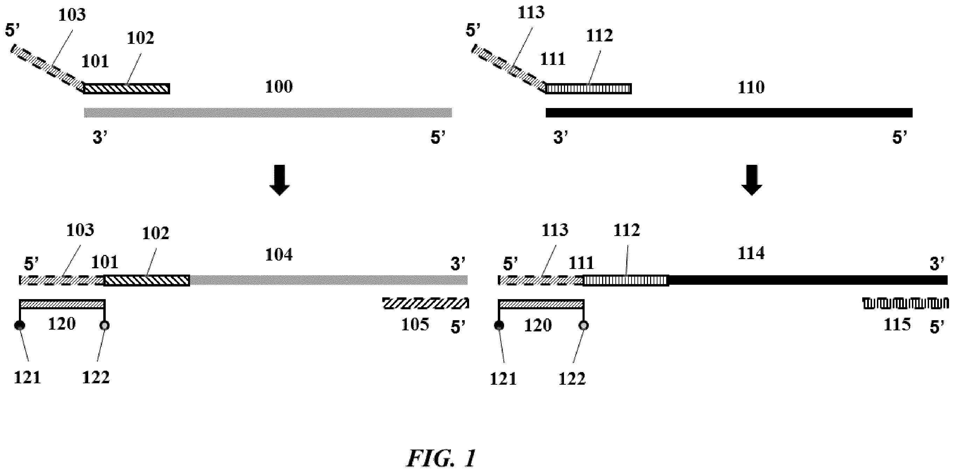

In one example, multiple sets of PCR primers are provided, each with specificity for a different target nucleic acid. One primer from each set (e.g., a forward primer) comprises a first region complementary to a unique target nucleic acid and a second region complementary to a single, universal probe (see FIG. 1). A sample potentially comprising one or more targets is subjected to thermal cycling under appropriate conditions for target amplification. If at least one target nucleic acid complementary to any of the provided primers is present, the second region of the primers is incorporated into the amplification product and the universal probe binds to this region, thereby generating a signal. The presence of a signal serves to identify the presence of at least one target in a sample.

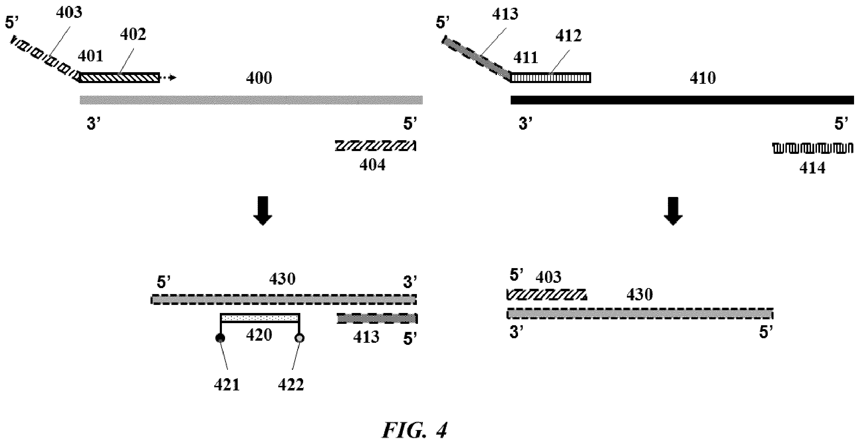

In another example, two sets of PCR primers are provided, each with specificity for one of two target nucleic acids. One primer from each set (e.g., a forward primer) comprises a first region complementary to a target nucleic acid and a second region complementary to a single, template nucleic acid (see FIG. 4). A sample potentially comprising the target nucleic acids is subjected to thermal cycling under appropriate conditions for target amplification. If both targets are present in the sample, the second region of the two primers is released via enzymatic digestion. Once released, the second regions bind to and enable amplification of the template nucleic acid (i.e., act as forward and reverse primers). A reporter probe complementary to the template nucleic acid is provided. Amplification of the template nucleic acid in the presence of the reporter probe generates a signal. The presence of a signal may serve to identify the presence of both targets in a sample.

### Definitions

The term "complementarity," or "complementary," as used herein, can refer to a property of a nucleic acid or the region of a nucleic acid wherein the nucleic acid is capable of binding, annealing, or otherwise attaching to another nucleic acid under a given set of conditions. A region of a nucleic acid which has complementarity to a region of another nucleic acid can comprise nucleotides which are complementary to those on the other nucleic acid. Complementarity may be of varying degrees. For example, a region of a nucleic acid can have between about 50% and about 100%, between about 60% and about 100%, between about 70% and about 100%, between about 80% and about 100%, between about 90% and about 100%, between about 95% and about 100%, between about 99% and about 100%, or about 100% complementarity to a region of another nucleic acid. Degrees of complementarity can describe the portion of a nucleic acid which is capable of attaching to another nucleic acid under appropriate conditions. Degrees of complementarity can describe the amount of a nucleic acid or region of a nucleic acid comprising nucleotides which are the complement of those on another nucleic acid. A nucleic acid with complementarity can be a double-stranded nucleic acid. A nucleic acid with complementarity can be a single-stranded nucleic acid. A single-stranded nucleic acid which is complementary to another single-stranded nucleic acid can bind to a region of another single-stranded nucleic acid under appropriate conditions. Appropriate conditions for binding of one complementary nucleic acid to another can include appropriate buffer conditions and appropriate temperature conditions. Appropriate buffer conditions can include appropriate salt concentrations, such as concentrations of sodium salts, magnesium salts, or potassium salts. Appropriate temperature conditions can include an annealing temperature which is appropriate for binding of one nucleic acid to another. While a nucleic acid with complementarity to another nucleic acid may bind to the other nucleic acid under appropriate conditions, the same nucleic acid may not bind under inappropriate conditions, such as inappropriate salt concentrations or temperature conditions.

The term "homologous" or "homology", as used herein, can refer to a property of a nucleic acid or the region of a nucleic acid wherein the nucleic acid comprises nucleotides which are identical to those comprised in another nucleic acid. A nucleic acid which is homologous to another nucleic acid can comprise one or more nucleotides which are identical to those on the other nucleic acid. Homology may be of varying degrees, depending on the number of nucleotides which are identical. A region of a nucleic acid which is homologous to a region of another nucleic acid may have a certain degree of homology. For example, a region of a nucleic acid can have between about 50% and about 100%, between about 60% and about 100%, between about 70% and about 100%, between about 80% and about 100%, between about 90% and about 100%, between about 95% and about 100%, between about 99% and about 100%, or about 100% homology to a region of another nucleic acid.

The term "primer," as used herein, can refer to an oligonucleotide or nucleic acid which can bind to another nucleic acid and facilitate transcription and/or nucleic acid synthesis. A primer can be double-stranded. A primer can be single-stranded. A primer can be a forward primer or a reverse primer. A forward primer and a reverse primer can be those which bind to opposite strands of a double-stranded nucleic acid. For example, a forward primer can bind to a region of a first strand (e.g., Watson strand) derived from a nucleic acid, and a reverse primer can bind to a region of a second strand (e.g., Crick strand) derived from the nucleic acid. A forward primer may bind to a region closer to the start site of a gene relative to a reverse primer or may bind closer to the end site of a gene relative to a reverse primer. A forward primer may bind to the coding strand of a nucleic acid, or may bind to the non-coding strand of a nucleic acid. A reverse primer may bind to the coding strand of a nucleic acid, or may bind to the non-coding strand of a nucleic acid.

### Nucleic Acid Detection

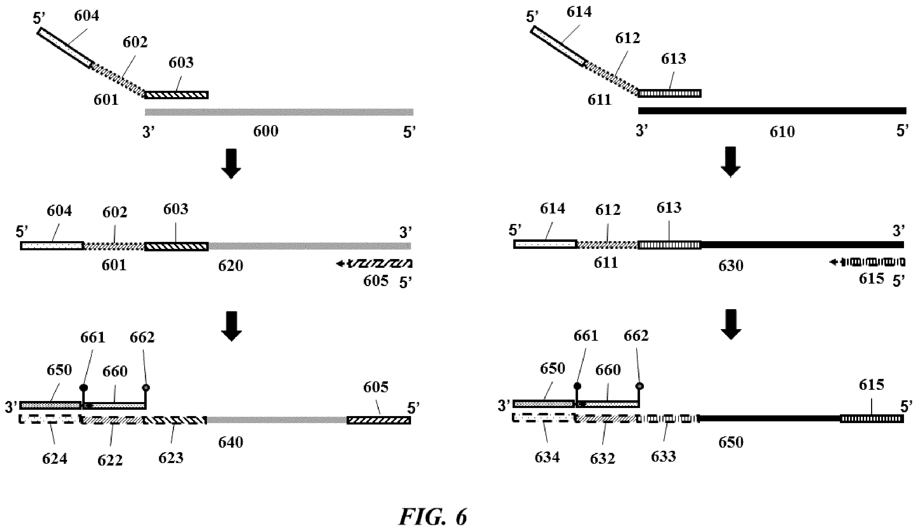

In a first broad aspect, the present disclosure provides methods for detecting the presence or absence of at least one target nucleic acid in a sample. The sample may comprise one or more target nucleic acids, or no target nucleic acids. Methods may comprise forming a mixture of the sample, a signal oligonucleotide probe, and multiple sets of oligonucleotide primers. FIG. 1 shows an example mixture comprising target nucleic acids 100 and 110, a signal oligonucleotide probe 120, and oligonucleotide primers 101, 111, 105, and 115. FIG. 6 shows another example mixture comprising target nucleic acids 600 and 610, a signal oligonucleotide probe 660, and oligonucleotide primers 601, 611, 605, and 615. Each set of oligonucleotide primers may comprise a forward oligonucleotide primer and a reverse oligonucleotide primer. Each oligonucleotide primer may comprise a region with complementarity to a target nucleic acid. FIG. 1 shows two example sets of oligonucleotide primers. A first set of oligonucleotide primers includes forward primer 101, comprising a region 102 with complementarity to a first target nucleic acid 100, and reverse primer 105. A second set of oligonucleotide primers includes forward primer 111, comprising a region 112 with complementarity to a second target nucleic acid 110, and reverse primer 115. FIG. 6 shows two additional example sets of oligonucleotide primers. A first set of oligonucleotide primers includes forward primer 601, comprising a region 603 with complementarity to a first target nucleic acid 600, and reverse primer 605. A second set of oligonucleotide primers includes forward primer 611, comprising a region 613 with complementarity to a second target nucleic acid 610, and reverse primer 615. Once formed, a mixture may be thermally cycled under conditions appropriate to amplify each target nucleic acid from the sample. Amplification may be performed by, for example, polymerase chain reaction (PCR). Subsequent to or simultaneous with thermal cycling, a signal may be generated if at least one target nucleic acid is present in the mixture. The signal may be detected, thereby detecting the presence or absence of at least one target nucleic acid in the sample.

Each forward oligonucleotide primer may comprise an additional region with complementarity to a signal oligonucleotide probe. FIG. 1 shows regions 103 and 113, each with complementarity to signal oligonucleotide probe 120. Under appropriate conditions, the first oligonucleotide primer may be incorporated into an amplification product of a target nucleic acid, which may be produced by a nucleic acid enzyme. FIG. 1 shows forward primer 101 incorporated into an amplification product 104 and forward primer 111 incorporated into an amplification product 114. Once incorporated, a signal oligonucleotide probe in a mixture may bind to the additional region of the forward oligonucleotide primer, thereby allowing for signal generation. FIG. 1 shows one copy of signal oligonucleotide probe 120 bound to region 103 of forward primer 103 and a second copy of signal oligonucleotide probe 120 bound to region 113 of forward primer 111. A signal oligonucleotide probe may comprise a signal tag. A signal tag may comprise a fluorophore and a quencher, which may act in the suppression and generation of a fluorescent signal. FIG. 1 shows signal probe 120 comprising a fluorophore 122 and a quencher 121. FIG. 6 shows a signal oligonucleotide probe comprising a fluorophore 662 and a quencher 661. A nucleic acid enzyme (e.g., nucleic acid polymerase) may be used to degrade the signal oligonucleotide probe and release the quencher, thereby generating a signal.

Alternatively or in addition, each forward oligonucleotide primer may comprise an additional region homologous to (i.e., with homology to) a signal oligonucleotide probe. FIG. 6 shows regions 602 and 612, each homologous to signal oligonucleotide probe 660. In some cases, a mixture may further comprise a universal primer. Each forward oligonucleotide primer may comprise a third region homologous to a universal primer. FIG. 6 shows regions 604 and 614, each complementary to universal primer 650. Under appropriate conditions, the first forward oligonucleotide primer may be incorporated into an amplification product of a target nucleic acid, which may be produced by a nucleic acid enzyme. FIG. 6 shows forward primer 601 incorporated into an amplification product 620 and forward primer 611 incorporated into an amplification product 630. Once incorporated, a reverse primer may be used to generate a reverse complement product. FIG. 6 shows reverse primers 605 and 615 used to generate reverse complement products 640 and 650 from products 620 and 630. Product 640 comprises reverse complement sequences 622, 623, and 624 generated from regions 602, 603, and 604. Product 650 comprises reverse complement sequences 632, 633, and 634 generated from regions 612, 613, and 614. Universal primer 650 may bind to sequences 624 and 634. The signal oligonucleotide probe 660 may bind to sequences 622 and 632. FIG. 6 shows one copy of a signal oligonucleotide probe 660 bound to sequence 622 of product 640 and a second copy of signal oligonucleotide probe 660 bound to sequence 632 of product 650. The universal primer may be used to amplify a reverse complement product, thereby allowing for signal generation, for example, by degrading the signal oligonucleotide probe to release the quencher.

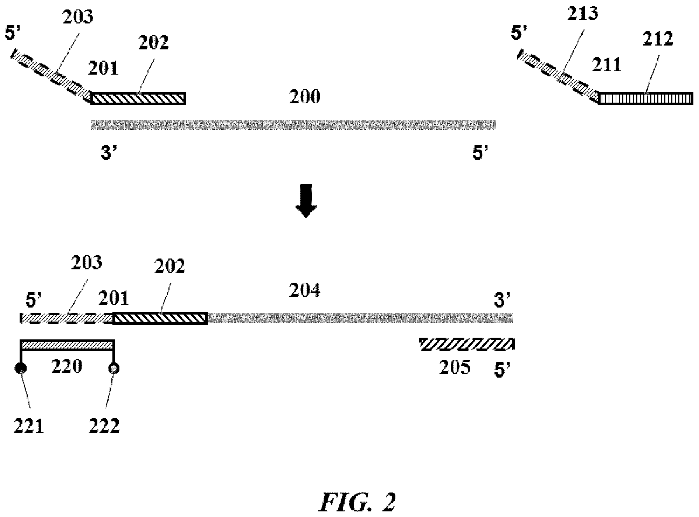

FIG. 2 shows another example mixture of the present disclosure comprising target nucleic acid 200, a first forward oligonucleotide primer 201 comprising a first region 202 with complementarity to the target nucleic acid 200, and a second region 203 with complementarity to a signal oligonucleotide probe 220. The mixture also comprises a second forward oligonucleotide primer 211 comprising a first region 212 with complementarity to a second member (not present in the mixture) and a second region 213 with complementarity to signal oligonucleotide probe 220. First forward oligonucleotide primer 201 is incorporated into amplification product 204. Signal oligonucleotide probe 220 comprising a quencher 221 and a fluorophore 222 binds to the second region 203 of the first forward oligonucleotide primer. A reverse oligonucleotide primer 205 is used to extend the first nucleic acid product 204 using a nucleic acid polymerase (not shown in FIG. 2). The signal oligonucleotide probe 220 is degraded by the exonuclease activity of the nucleic acid polymerase, thereby releasing the quencher 221 and generating a signal.

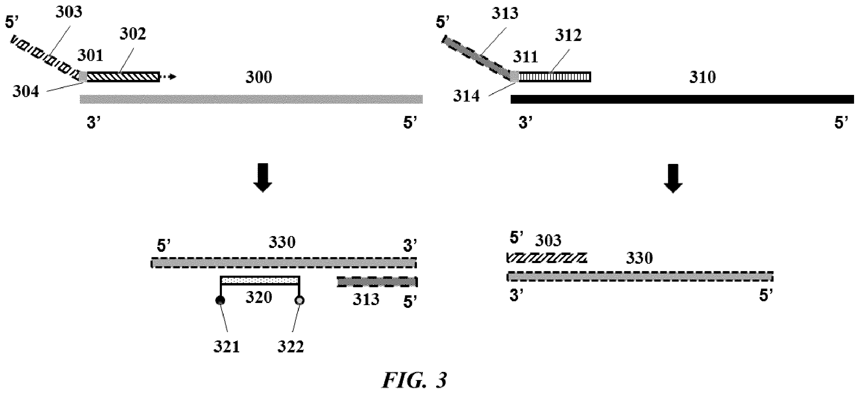

In another broad aspect, the present disclosure provides methods for detecting the presence or absence of two target nucleic acids in a sample. The sample may comprise one, both, or neither target nucleic acid. Methods may comprise forming a mixture of the sample, a template nucleic acid, a signal oligonucleotide probe, and two oligonucleotide primers. FIG. 3 shows an example mixture comprising target nucleic acids 300 and 310, a signal oligonucleotide probe 320, oligonucleotide primers 301 and 311, and a template nucleic acid 330. The signal oligonucleotide probe may be complementary to the template nucleic acid. Each oligonucleotide primer may comprise a region with complementarity to one of the two target nucleic acids. This region may comprise one or more non-natural nucleotides. FIG. 3 shows two example primers. Primer 301 comprises a region 302 with complementarity to target nucleic acid 300. Primer 311 comprises a region 312 with complementarity to target nucleic acid 310.Once formed, a mixture may be subjected to conditions sufficient to anneal the oligonucleotide primers to their complementary target nucleic acids, as shown in FIG. 3. These conditions may be such that the primers are degraded. A mixture may be thermally cycled under conditions appropriate to amplify the template nucleic acid. Amplification may be performed by, for example, polymerase chain reaction (PCR). Subsequent to or simultaneous with thermal cycling, a signal may be generated if both target nucleic acids are present in the mixture. The signal may be detected, thereby detecting the presence or absence of two target nucleic acids in the sample.

The region of each oligonucleotide primer with complementarity to a target nucleic acid may comprise one or more non-natural nucleotides. A non-natural nucleotide may be, for example, deoxyinosine. FIG. 3 shows a primer 301 comprising a non-natural nucleotide 304 and a primer 311 comprising a non-natural nucleotide 314. Each oligonucleotide primer may comprise an additional region with complementarity to a template nucleic acid. FIG. 3 shows regions 303 and 313, each with complementarity to a template nucleic acid 330. Under appropriate conditions, the region of the oligonucleotide primers with complementarity to a target nucleic acid may anneal to the target, as shown in FIG. 3. Conditions may be such that the region with complementarity to the template nucleic acid does not anneal to the target. FIG. 3 shows regions 302 and 312 annealed to target nucleic acids 300 and 310. Once annealed, the primers may be degraded by, for example, a nucleic acid enzyme. A nucleic acid enzyme may be capable of degrading a non-natural nucleotide. The nucleic acid enzyme may be an endonuclease V such as, for example, an E. coli endonuclease V. Primer degradation may release the additional region with complementarity to the template nucleic acid. The regions from the two primers may anneal to the template nucleic acid and, under appropriate conditions, serve to amplify the template nucleic acid (e.g., may act as forward and reverse primers). A signal oligonucleotide probe may bind to the template nucleic acid. FIG. 3 shows signal probe 320 bound to template nucleic acid 330. A signal oligonucleotide probe may comprise a fluorophore and a quencher, which may act in the suppression or generation of a fluorescent signal. A nucleic acid enzyme (e.g., nucleic acid polymerase) may be used to degrade the signal oligonucleotide probe and release the quencher, thereby generating a signal.

In another broad aspect, the present disclosure provides methods for detecting the presence or absence of two target nucleic acids in a sample. The sample may comprise one, both, or neither target nucleic acid. Methods may comprise forming a mixture of the sample, a template nucleic acid, a signal oligonucleotide probe, and two sets of oligonucleotide primers. FIG. 4 shows an example mixture comprising target nucleic acids 400 and 410, a signal oligonucleotide probe 420, oligonucleotide primers 401, 404, 411, and 414, and a template nucleic acid 430. Each set of oligonucleotide primers may comprise a forward oligonucleotide primer and a reverse oligonucleotide primer. The signal oligonucleotide probe may have complementarity to the template nucleic acid. Each oligonucleotide primer may comprise a region with complementarity to one of the two target nucleic acids. FIG. 4 shows two example primers. Primer 401 comprises a region 402 with complementarity to target nucleic acid 400. Primer 411 comprises a region 412 with complementarity to target nucleic acid 410. Once formed, a mixture may be thermally cycled under conditions sufficient to amplify the target nucleic acids. These conditions may be such that the primers are degraded. A mixture may be thermally cycled under conditions appropriate to amplify the template nucleic acid. Amplification may be performed by, for example, polymerase chain reaction (PCR). Subsequent to or simultaneous with thermal cycling, a signal may be generated if both target nucleic acids are present in the mixture. The signal may be detected, thereby detecting the presence or absence of two target nucleic acids in the sample.

Each oligonucleotide primer may comprise an additional region with complementarity to a template nucleic acid. FIG. 4 shows regions 403 and 413, each with complementarity to template nucleic acid 430. Under appropriate conditions, the region of the oligonucleotide primers with complementarity to a target nucleic acid may anneal to the target, as shown in FIG. 4. Conditions may be such that the region with complementarity to the template nucleic acid does not anneal to the target. FIG. 4 shows regions 402 and 412 annealed to target nucleic acids 400 and 410. Once annealed, the target nucleic acids may be amplified. During amplification, the oligonucleotide primers may be degraded, releasing the regions complementary to the template nucleic acid. Primers may be degraded by the endonuclease activity of a nucleic acid polymerase. The regions from the two primers may anneal to the template nucleic acid and, under appropriate conditions, serve to amplify the template nucleic acid (e.g., may act as forward and reverse primers). A signal oligonucleotide probe may bind to the template nucleic acid. FIG. 4 shows signal probe 420 bound to template nucleic acid 430. A signal oligonucleotide probe may comprise a fluorophore and a quencher, which may act in the suppression or generation of a fluorescent signal. A nucleic acid enzyme (e.g., nucleic acid polymerase) may be used to degrade the signal oligonucleotide probe and release the quencher, thereby generating a signal.

### Oligonucleotide Probes

Mixtures and compositions of the present disclosure may comprise a signal oligonucleotide probe. The concentration of a signal oligonucleotide probe may be such that it is in excess relative to other components in the mixture. A mixture may comprise more than one signal oligonucleotide probe. Multiple signal oligonucleotide probes may be the same, or may be different. In some examples, a mixture comprises a first signal oligonucleotide probe and one or more additional signal oligonucleotide probes. An additional signal oligonucleotide probe may be an xth signal oligonucleotide probe. x may be any number which describes a signal oligonucleotide probe as one of multiple signal oligonucleotide probes. x may be an integer greater than one. x may be at least 2, 3, 4, 5, 6, 7, 8, 9, 10, 11, 12, 13, 14, 15, 20, or higher, x may be at most 20, 15, 14, 13, 12, 11, 10, 9, 8, 7, 6, 5, 4, 3, or 2. In one example, x may be 2, such that an xth signal oligonucleotide probe is a 2nd signal oligonucleotide probe. A signal oligonucleotide probe may be a nucleic acid (e.g., DNA, RNA, etc.). A signal oligonucleotide probe may be at least 5, 10, 15, 20, 30, or 50 nucleotides in length, or more. A signal oligonucleotide probe may be at most 50, 30, 20, 15, 10 or, 5, nucleotides in length. A signal oligonucleotide probe may have less than 50%, 40%, 30%, 20%, 10%, 5%, or 1% complementarity to any member of the plurality of target nucleic acids. A signal oligonucleotide probe may have no complementarity to any member of a plurality of target nucleic acids (i.e. no complementarity to any target nucleic acid). A signal oligonucleotide probe may comprise a signal tag. A signal tag may comprise a means of generating a signal such as, for example, a fluorophore. A fluorophore may be, for example, FAM, TET, HEX, JOE, Cy3, or Cy5. A signal tag may further comprise one or more quenchers. A quencher may inhibit signal generation from a fluorophore. A quencher may be, for example, TAMRA, BHQ-1, BHQ-2, or Dabcyl.

### Oligonucleotide Primers

An oligonucleotide primer of the present disclosure may be a deoxyribonucleic acid. An oligonucleotide primer may be a ribonucleic acid. An oligonucleotide primer may comprise one or more non-natural nucleotides. A non-natural nucleotide may be, for example, deoxyinosine. An oligonucleotide primer may be a forward primer. An oligonucleotide primer may be a reverse primer. An oligonucleotide primer may be between about 5 and about 100 nucleotides in length. An oligonucleotide primer may be at least 5, 10, 11, 12, 13, 14, 15, 16, 17, 18, 19, 20, 25, 30, 35, 40, 45, 50, 60, 70, 80, 90, or 100 base pairs in length, or more. An oligonucleotide primer may be at most 100, 90, 80, 70, 60, 50, 45, 40, 35, 30, 25, 20, 19, 18, 17, 16, 15, 14, 13, 12, 11, 10, or 5 nucleotides in length.

An oligonucleotide primer may comprise one or more regions. In some cases, an oligonucleotide primer has two regions. In some cases, an oligonucleotide primer has three regions. A region may be between about 5 and about 50 nucleotides in length. A region may be at least 5, 10, 11, 12, 13, 14, 15, 16, 17, 18, 19, 20, 25, 30, 35, 40, 45, or 50 nucleotides in length, or more. A region may be at most 50, 45, 40, 35, 30, 25, 20, 19, 18, 17, 16, 15, 14, 13, 12, 11, 10, or 5 nucleotides in length. A region may have complementarity to a region of a target nucleic acid. A region may have complementarity to a signal oligonucleotide probe. A region may be homologous to a signal oligonucleotide probe. A region may have complementarity to a universal primer. A region may be homologous to a universal primer. A region with complementarity or homology to a universal primer region may have little or no complementarity or homology to either a region of a target nucleic acid or a signal oligonucleotide probe. A region may have complementarity to a region of a template nucleic acid. A first region of a first forward oligonucleotide primer may be 5' relative to a second region of the first forward oligonucleotide primer. A first region of a second forward oligonucleotide primer may be 5' relative to a second region of the second forward oligonucleotide primer. A third region of a first forward oligonucleotide primer may be 5' relative to a first and second region of the first forward oligonucleotide primer. A third region of a second forward oligonucleotide primer may be 5' relative to a first and second region of the second forward oligonucleotide primer.

Mixtures of the present disclosure may comprise one or more oligonucleotide primers. An oligonucleotide primer may be an nth oligonucleotide primer. n may be any number which describes an oligonucleotide primer as one of multiple oligonucleotide primers. n may be an integer greater than two. n may be at least 3, 4, 5, 6, 7, 8, 9, 10, 15, 20, or higher, n may be at most 20, 15, 10, 9, 8, 7, 6, 5, 4, or 3. In one example, n may be 3, such that an nth oligonucleotide primer is a 3rd oligonucleotide primer. An oligonucleotide primer may be a yth oligonucleotide primer. y may be any number which describes an oligonucleotide primer as one of multiple oligonucleotide primers. y may be an integer greater than n. y may be at least 4, 5, 6, 7, 8, 9, 10, 11, 15, 20, or higher. y may be at most 20, 15, 10, 9, 8, 7, 6, 5, or 4. In one example, y may be 4, such that a yth oligonucleotide primer is a 4th oligonucleotide primer.

### Nucleic Acid Enzymes

Mixtures and compositions of the present disclosure may comprise one or more nucleic acid enzymes. A nucleic acid enzyme may have exonuclease activity. A nucleic acid enzyme may have endonuclease activity. A nucleic acid enzyme may have RNase activity. A nucleic acid enzyme may be capable of degrading a nucleic acid comprising one or more ribonucleotide bases. A nucleic acid enzyme may be, for example, RNase H or RNase III. An RNase III may be, for example, Dicer. A nucleic acid may be an endonuclease I such as, for example, a T7 endonuclease I. A nucleic acid enzyme may be capable of degrading a nucleic acid comprising a non-natural nucleotide. A nucleic acid enzyme may be an endonuclease V such as, for example, an *E. coli* endonuclease V. A nucleic acid enzyme may be a polymerase (e.g., a DNA polymerase). A polymerase may be a Taq polymerase. A nucleic acid enzyme may be capable, under appropriate conditions, of degrading a signal oligonucleotide probe. A nucleic acid enzyme may, for example, be capable of releasing a quencher from a signal oligonucleotide probe by exonuclease activity.

### Thermal Cycling

Methods of the present disclosure may comprise thermal cycling. Thermal cycling may comprise one or more thermal cycles. Thermally cycling may be performed under reaction conditions appropriate to amplify one or more nucleic acids (e.g., one or more members of a plurality of target nucleic acids, one or more target nucleic acids, etc.). Amplification may comprise, for example, polymerase chain reaction (PCR). Appropriate reaction conditions may include appropriate temperature conditions, appropriate buffer conditions, and the presence of appropriate reagents. Appropriate temperature conditions may be such that each thermal cycle is performed at a desired annealing temperature. A desired annealing temperature may be sufficient for annealing of a region of an oligonucleotide primer to a target nucleic acid. A desired annealing temperature may be sufficient for annealing of a region of an oligonucleotide primer to a signal oligonucleotide probe. A desired annealing temperature may be insuffucieent for a region (e.g., a region with complementarity to a template nucleic acid) to anneal to a target nucleic acid. Appropriate buffer conditions may be such that the appropriate salts are present in a buffer used during thermal cycling. Appropriate salts may include magnesium salts, potassium salts, ammonium salts. Appropriate buffer conditions may be such that the appropriate salts are present in appropriate concentrations. Appropriate reagents for amplification of each member of a plurality of target nucleic acids with PCR may include deoxytriphosphates (dNTPs). dNTPs may comprise natural or non-natural dNTPs including, for example, dATP, dCTP, dGTP, dTTP, dUTP, and variants thereof.

In some aspects, a mixture may be thermally cycled under reaction conditions appropriate to amplify a template nucleic acid. Amplification may comprise, for example, PCR. Amplification of a template nucleic acid may require binding or annealing of both a region released from a first oligonucleotide and a region released from a second oligonucleotide primer to the template nucleic acid. A region released from an oligonucleotide primer may enable amplification of a template nucleic acid by acting as a forward and reverse primer for PCR amplification.

### Signal Generation and Detection

Methods of the present disclosure may be such that one or more signal oligonucleotide probes are degraded by a nucleic acid enzyme. A signal oligonucleotide probe may be degraded by the exonuclease activity of a nucleic acid enzyme. A signal oligonucleotide probe may generate a signal upon degradation. In some cases, a signal oligonucleotide probe may generate a signal only if at least one member of a plurality of target nucleic acids is present in a mixture. For example, a signal oligonucleotide probe may be degraded only when bound (i.e., annealed) to a region of an oligonucleotide primer (e.g., a second region of a forward oligonucleotide primer). In some cases, a signal oligonucleotide probe may generate a signal only if both a first and second member of a plurality of target nucleic acids is present in a mixture. For example, a signal oligonucleotide probe may be degraded only when bound (i.e. annealed) to a region of a template nucleic acid. In cases where a signal oligonucleotide probe comprises a signal tag, the signal oligonucleotide probe may be degraded when bound to a region of an oligonucleotide primer, thereby generating a signal. A signal may be a fluorescent signal. A signal tag may comprise a quencher and a fluorophore, such that the quencher is released from a signal tag upon degradation of a signal oligonucleotide probe, thereby generating a fluorescent signal. Thermal cycling of the present disclosure may generate one signal, or more than one signal. Thermal cycling may generate at least 1, 2, 3, 4, 5, 6, 7, 8, 9, 10 signals, or more. Thermal cycling may generate at most 10, 9, 8, 7, 6, 5, 4, 3, 2, or 1 signal. Multiple signals may be of the same type or of different types. Signals of different types may be fluorescent signals with different fluorescent wavelengths. Signals of different types may be generated by signal tags comprising different fluorophores. Signals of the same type may be of different intensities (e.g., different intensities of the same fluorescent wavelength). Signals of the same type may be generated by signal tags comprising the same fluorophore. Signal tags comprising the same fluorophore may generate different signals by nature of being at different concentrations, thereby generating different intensities of the same signal type.

The presence or absence of one or more signals may be detected. One signal may be detected, or multiple signals may be detected. Multiple signals may be detected simultaneously. Alternatively, multiple signals may be detected sequentially. A signal may be detected by any means, including, for example, detection of a fluorescent signal. A signal may be detected throughout the process of thermal cycling, for example, at the end of each thermal cycle. In some cases, the signal intensity increases with each thermal cycle. The signal intensity may increase in a sigmoidal fashion.

The presence of a signal may be correlated to the presence of at least one member of a plurality of target nucleic acids. Alternatively, the presence of a signal may be correlated to the presence of both a first and second member of a plurality of target nucleic acids. Correlating the presence of a signal to the presence of one or more target nucleic acids may comprise establishing a signal intensity threshold. A signal intensity threshold may be different for different signals. Making a correlation may comprise determining whether the intensity of a signal increases beyond a signal intensity threshold. The presence of a signal may be correlated with the presence of at least one of all members of a plurality of target nucleic acids. The presence of a first signal may be correlated with the presence of at least one of a first subset of members of a plurality of target nucleic acids, and the presence of a second signal may be correlated with the presence of at least one of a second subset of members of a plurality of target nucleic acids. Multiple signals may be detected, and the presence of each may be correlated with the presence of at least one of a subset of members of a plurality of target nucleic acids. The presence of least 1, 2, 3, 4, 5, 6, 7, 8, 9, 10, or more signals may be correlated with the presence of at least one of at least 1, 2, 3, 4, 5, 6, 7, 8, 9, 10, or more subsets of members of a plurality of target nucleic acid molecules.

The absence of a signal may be correlated with the absence of each of at least a subset of members of a plurality of target nucleic acids. Alternatively, the absence of a signal may be correlated with the absence of either a first or a second member of a plurality of target nucleic acids. The absence of a signal may be correlated with the absence of each member of the plurality of target nucleic acids. The absence of a first signal may be correlated with the absence of each of a first subset of members of a plurality of target nucleic acids, and the absence of a second signal may be correlated with the absence of each of a second subset of members of a plurality of target nucleic acids. Multiple signals may be detected, and the absence of each may be correlated with the absence of each of a subset of members of a plurality of target nucleic acids. The absence of least 1, 2, 3, 4, 5, 6, 7, 8, 9, 10, or more signals may be correlated with the absence of each of at least 1, 2, 3, 4, 5, 6, 7, 8, 9, 10, or more subsets of members of a plurality of target nucleic acid molecules.

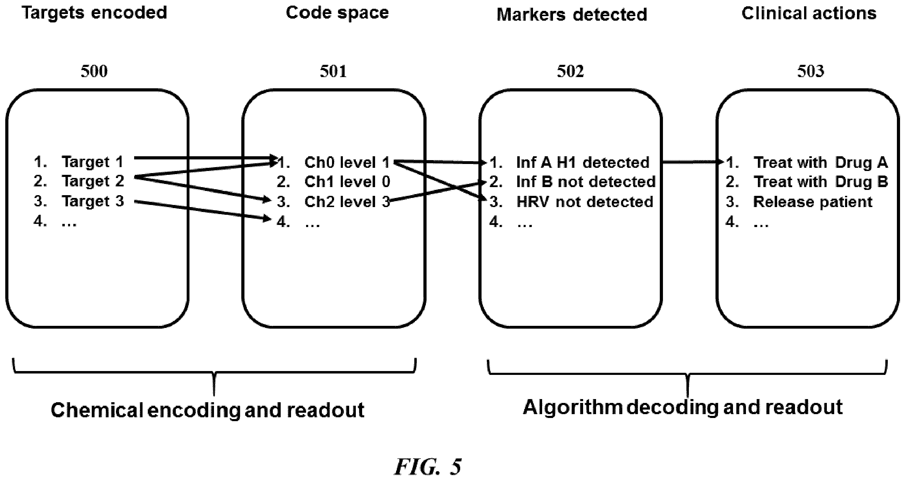

Detection and analysis of one or more signals may be useful in, for example, determining the presence or absence of a large number of targets in a sample. Analysis of these methods may be useful in clinical applications, where detection of one or more targets in a subject may inform clinical decisions. FIG. 5 shows a schematic of an example analysis that may be performed. Target nucleic acids are provided as described herein in operation 500. Nucleic acids are detected, generating fluorescent readouts in operation 501. In operation 502, analysis of the fluorescent readouts results in the determination of the presence or absence of a given viral pathogen based on the presence or absence of one or more targets. Based on the presence or absence of one or more viral pathogens, clinical actions are taken in operation 503, such as treatment of a subject with a clinically appropriate drug.

### Samples and Targets

Target nucleic acids of the present disclosure may be derived from any source including, for example, viruses, bacterial cells, and eukaryotic cells. Target nucleic acids may be derived from one or more cells. A cell may be a tumor cell. A cell may be a cell suspected of comprising a viral pathogen. In some cases, target nucleic acids are derived from a cell-free sample (e.g., plasma). Target nucleic acids may be cell-free nucleic acid. Cell-free nucleic acid may be, for example, cell-free tumor DNA, cell-free fetal DNA, cell-free RNA, etc. Target nucleic acids may comprise deoxyribonucleic acid (DNA). Target nucleic acids may comprise ribonucleic acid (RNA). RNA may be any kind of RNA, including messenger RNA, transfer RNA, ribosomal RNA, and microRNA. RNA may be viral RNA. A target nucleic acid sample may comprise one or more members (e.g., may comprise one or more target nucleic acids). A member may be any region of a target nucleic acid. A member may be of any length. A member may be, for example, up to 1, 2, 3, 4, 5, 10, 20, 50, 100, 500, 1000, 5000, 10000, 50000, 100000 nucleotides in length, or more. In some instances, a member may be a gene. A member may be any region of a target nucleic acid which is of interest. For example, a member may be a gene whose detection may be useful in diagnosing one or more diseases. A gene may be a viral gene or bacterial gene whose detection may be useful in identifying the presence or absence of a pathogen in a subject.

### Template Nucleic Acids

The present disclosure provides, in some aspects, one or more template nucleic acids. A template nucleic acid may be added to a sample as described herein. A template nucleic acid may be useful in, for example, ensuring the generation of a signal only if both a first and second target nucleic acid are present in a sample, thereby verifying the presence of both targets in a single sample using a single signal. A template nucleic acid may comprise DNA, RNA, or a combination thereof. A template nucleic acid may comprise a first region, a second region, and a third region. The first region may be 3' relative to the second region. The first region may be 5' relative to the third region. A template nucleic acid may be derived from any source (i.e. a natural source). A template nucleic acid may be synthetically generated, for example, by chemical synthesis.

### Kits

Also provided herein are kits for detecting the presence or absence of at least one member of a plurality of target nucleic acids in a sample. Kits may be used for detecting the presence of a first and second member of a plurality of target nucleic acids in a sample. Kits may include one or more signal oligonucleotide probes. Signal oligonucleotide probes may be lyophilized. Signal oligonucleotide probes may be present at different concentrations. Signal oligonucleotide probes may comprise a signal tag, which may comprise, for example, a fluorophore and one or more quenchers. Kits may include one or more oligonucleotide primers as described herein, including, for example, a first oligonucleotide primer (e.g., a first forward oligonucleotide primer), a second oligonucleotide primer (e.g., a second forward oligonucleotide primer), a third oligonucleotide primer (e.g., a first reverse oligonucleotide primer), and/or a fourth oligonucleotide primer (e.g., a second reverse oligonucleotide primer). The oligonucleotide primers may comprise various regions as disclosed herein. One or more of the oligonucleotide primers may be lyophilized. In some cases, all of the oligonucleotide primers may be lyophilized. Kits may comprise one or more nucleic acid enzymes. A nucleic acid enzyme may be a nucleic acid polymerase. A nucleic acid polymerase may be a deoxyribonucleic acid polymerase. A nucleic acid enzyme may be an RNase. An RNase may be an RNase III. An RNase III may be Dicer. The nucleic acid enzyme may be an endonuclease. An endonuclease may be an endonuclease I. An endonuclease I may be a T7 endonuclease I. Kits may comprise instructions for using any of the foregoing in the methods described herein.

### EXAMPLES

### Example 1

A biological sample is obtained from a subject suspected of having human immunodeficiency virus (HIV) and/or hepatitis C virus (HCV). The sample is mixed with a TaqMan signal oligonucleotide probe, two sets of forward and reverse primers, and a Taq DNA polymerase.

The first forward primer comprises two regions: a first region complementary to HIV and a second region complementary to the signal oligonucleotide probe. The first reverse primer is complementary to HIV. The second forward primer comprises two regions, a first region complementary to a region of HCV and a second region complementary to the signal oligonucleotide probe. The second reverse primer is complementary to HCV.

The mixture is thermally cycled under appropriate buffer conditions and temperature conditions, and comprising the appropriate reagents, such that the primers anneal to their respective targets, which are amplified by polymerase chain reaction. During this process, the regions complementary to the signal oligonucleotide probe are incorporated into the amplification product. The signal oligonucleotide probe binds to the amplification products by annealing to the complementary regions from the forward primers. The Taq polymerase degrades the signal oligonucleotide probes via its exonuclease activity, thus releasing the quencher. A fluorescent signal is generated by the fluorophore upon release of the quencher. The fluorescent signal is detected, indicating the sample to contain HIV, HCV, or both.

### Example 2

A biological sample is obtained from a subject suspected of having human immunodeficiency virus (HIV), hepatitis C virus (HCV), hepatitis B virus (HBV), and/or hepatitis A virus. The sample is mixed with two different TaqMan signal oligonucleotide probes, four sets of forward and reverse primers, and a Taq DNA polymerase.

The first forward primer comprises two regions: a first region complementary to HIV and a second region complementary to the first signal oligonucleotide probe. The first reverse primer is complementary to HIV. The second forward primer comprises two regions, a first region complementary to a region of HCV and a second region complementary to the first signal oligonucleotide probe. The second reverse primer is complementary to HCV. The third forward primer comprises two regions, a first region complementary to a region of HBV and a second region complementary to the second signal oligonucleotide probe. The third reverse primer is complementary to HBV. The fourth forward primer comprises two regions, a first region complementary to a region of HCV and a fourth region complementary to the second signal oligonucleotide probe. The second reverse primer is complementary to HCV.

The mixture is thermally cycled under appropriate buffer conditions and temperature conditions, and comprising the appropriate reagents, such that the primers anneal to their respective targets, which are amplified by polymerase chain reaction. During this process, the regions complementary to the signal oligonucleotide probes are incorporated into the amplification product. The signal oligonucleotide probes binds to the amplification products by annealing to the respective complementary regions from the forward primers. The Taq polymerase degrades the signal oligonucleotide probes via its exonuclease activity, thus releasing the quencher. A fluorescent signal of a given intensity is generated by the fluorophores upon release of the quencher. The fluorescent signal generated from both probes is detected, indicating the sample to contain HIV, HCV, or both and HBV, HAV, or both.

### Example 3.

A biological sample is obtained from a subject suspected of having human immunodeficiency virus (HIV) and hepatitis C virus (HCV). The sample is mixed with a TaqMan signal oligonucleotide probe, two sets of forward and reverse primers, a chemically synthesized template nucleic acid, and a Taq DNA polymerase.

The signal oligonucleotide probe is complementary to the template nucleic acid. The first forward primer comprises two regions: a first region complementary to HIV and a second region complementary to the template nucleic acid. The first reverse primer is complementary to HIV. The second forward primer comprises two regions, a first region complementary to a region of HCV and a second region complementary to the template nucleic acid. The second reverse primer is complementary to HCV.

The mixture is thermally cycled under appropriate buffer conditions and temperature conditions, and comprising the appropriate reagents, such that the primers anneal to their respective targets, which are amplified by polymerase chain reaction.. The Taq polymerase releases the second region of the first forward oligonucleotide primer and the second region of the second oligonucleotide primer via its endonuclease activity. The second region of the first forward oligonucleotide primer and the second region of the second oligonucleotide primer are used as forward and reverse primers to amplify the template nucleic acid by thermally cycling the mixture under reaction conditions sufficient for template nucleic acid amplification. The signal oligonucleotide probe binds to the template nucleic acid. The Taq polymerase degrades the signal oligonucleotide probe via its exonuclease activity, thus releasing the quencher. A fluorescent signal is generated by the fluorophore upon release of the quencher. The fluorescent signal is detected, indicating the presence of both HIV and HCV in the sample.

### Example 4

Synthetic DNA comprising a sequence from Influenza A (FluA) or Influenza B (FluB) was generated. Three mixtures were generated comprising either 10⁵ copies of FluA alone, 10⁵ copies of FluB alone, or 10⁵ copies of both FluA and FluB. To each of these mixtures, primer pairs were added with regions complementary to either FluA or FluB. Each of the first forward primers from the primer pairs comprised, in addition to a region complementary to a target, a region complementary to a signal oligonucleotide probe (universal probe). A signal oligonucleotide probe (universal probe) was also added to each mixture. Table 1 shows the concentration added for each primer and the universal probe. Finally, reagents necessary to perform polymerase chain reaction were added.

**Table 1: Reaction components**

| Reagent | Concentration |
|---|---|
| FluA forward primer with reverse complement of universal probe | 1OnM |
| FluA reverse primer | 100nM |
| FluB forward primer with reverse complement of universal probe | 1OnM |
| FluB reverse primer | 100nM |
| Universal probe comprising a FAM fluorophore | 50nM |

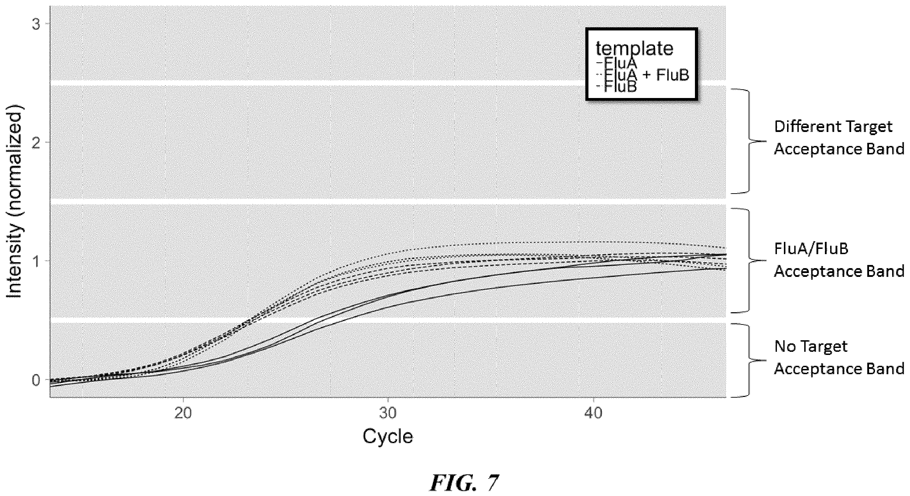

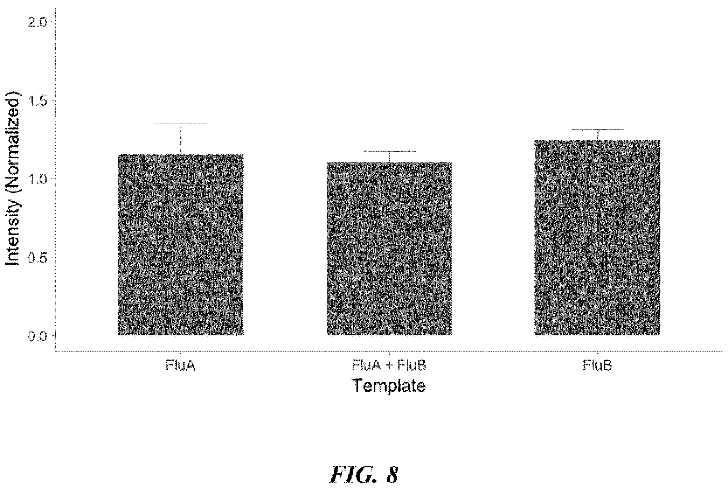

Each mixture was subjected to thermal cycling under conditions appropriate to amplify each target nucleic acid with polymerase chain reaction using an Applied Biosystems 7500 Fast Real-Time PCR system. Each mixture was subjected to 60 seconds at 95°C, followed by a series of forty-five thermal cycles each consisting of 30 seconds at 95°C followed by 120 seconds at 57°C. Fluorescence was measured at the end of each thermal cycle, and is plotted in FIG. 7. FIG. 7 shows that amplification and measurement of signals from each of the three mixtures results in a final fluorescence intensity in the same region ("FluA/FluB Acceptance Band"). FIG. 8 shows a bar graph of peak fluorescence intensity from FIG. 7. Error bars indicate 95% confidence bounds. Following fluorescence measurement, the presence of a signal in the mixtures was correlated to the presence of either or both of FluA or FluB in the mixture.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.
The following section of the description consists of numbered paragraphs simply providing statements of the invention already described herein. The numbered paragraphs in this section are not claims. The claims are set forth below in the later section headed "claims".
1. A method of detecting the presence or absence of at least one member of a plurality of target nucleic acids in a sample, the method comprising:
   (a) providing a sample comprising, or potentially comprising, at least one member of the plurality of target nucleic acids;
   (b) forming a mixture comprising the sample and
      (i) a signal oligonucleotide probe;
      (ii) a first forward oligonucleotide primer comprising: a first region complementary to a first region of a first member of the plurality of target nucleic acids; and a second region complementary or homologous to the signal oligonucleotide probe;
      (iii) a first reverse oligonucleotide primer comprising a region complementary to a second region of the first member of the plurality of target nucleic acids;
      (iv) a second forward oligonucleotide primer comprising: a first region complementary to a first region of a second member of the plurality of target nucleic acids; and a second region complementary or homologous to the signal oligonucleotide probe; and
      (v) a second reverse oligonucleotide primer comprising a region complementary to a second region of the second member of the plurality of target nucleic acids;
   (c) thermally cycling the mixture under conditions appropriate to amplify each member of the plurality of target nucleic acids with polymerase chain reaction (PCR), such that the signal oligonucleotide probe is degraded and a signal is generated if at least one member of the plurality of target nucleic acids is present in the mixture; and
   (d) detecting the presence or absence of the signal.
2. The method of paragraph 1, wherein the mixture further comprises a nucleic acid enzyme.
3. The method of paragraphs 1 or 2, wherein the signal oligonucleotide probe comprises a signal tag.
4. The method of paragraph 3, wherein, in (c), the signal is generated by the signal tag.
5. The method of paragraph 4, wherein the signal tag generates the signal upon degradation of the signal oligonucleotide probe.
6. The method of any of paragraphs 1-5, wherein, in (c), each thermal cycle is performed at an annealing temperature appropriate for annealing of the first region of the first forward oligonucleotide primer to the first target nucleic acid and annealing of the second region of the first forward oligonucleotide primer to the signal oligonucleotide probe.
7. The method of any of paragraphs 1-6, further comprising correlating the presence of the signal to the presence of at least one member of the plurality of target nucleic acids or correlating the absence of the signal with the absence of each member of the plurality of target nucleic acids.
8. A method of detecting the presence or absence of at least one member of a plurality of target nucleic acids in a sample, the method comprising:
   (a) providing a sample comprising, or potentially comprising, at least one member of the plurality of target nucleic acids;
   (b) forming a mixture of the sample and
      (i) a signal oligonucleotide probe;
      (ii) a first forward oligonucleotide primer comprising a region with complementarity to a first member of the plurality of target nucleic acids;
      (iii) a first reverse oligonucleotide primer comprising a region with complementarity to the first member of the plurality of target nucleic acids;
      (iv) a second forward oligonucleotide primer comprising a region with complementarity to a second member of the plurality of target nucleic acids; and
      (v) a second reverse oligonucleotide primer comprising a region with complementarity to the second member of the plurality of target nucleic acids;
   (c) thermally cycling the mixture under conditions appropriate to amplify each member of the plurality of target nucleic acids with polymerase chain reaction (PCR) and generating a signal if at least one member of the plurality of target nucleic acids is present in the mixture; and
   (d) detecting the presence or absence of the signal.
9. The method of paragraph 8, wherein the mixture further comprises a nucleic acid enzyme.
10. The method of paragraph 8 or 9, wherein the first forward oligonucleotide primer further comprises an additional region with complementarity or homology to the signal oligonucleotide probe.
11. The method of any of paragraphs 8-10, wherein the second forward oligonucleotide primer further comprises an additional region with complementarity or homology to the signal oligonucleotide probe.
12. The method of paragraph 11, wherein, in (c), each thermal cycle is performed at an annealing temperature appropriate for annealing of the first region of the second forward oligonucleotide primer to the second member of the plurality of target nucleic acids and annealing of the additional region of the second forward oligonucleotide primer to the signal oligonucleotide probe.
13. The method of any of paragraphs 8-12, wherein the mixture formed at (b) further comprises:
   (a) an nth forward oligonucleotide primer comprising: a first region with complementarity to an nth member of the plurality of target nucleic acids; and
   (b) an nth reverse oligonucleotide primer comprising a region with complementarity to a region of an nth member of the plurality of target nucleic acids;
   wherein n is an integer greater than 2.
14. The method of paragraph 13, wherein the nth forward oligonucleotide primer further comprises an additional region with complementarity to the signal oligonucleotide probe.
15. The method of paragraph 13 or 14;
   wherein the mixture formed at (b) further comprises:
      (a) an xth signal oligonucleotide probe;
      (b) a yth forward oligonucleotide primer comprising a first region with complementarity to a yth member of the plurality of target nucleic acids; and
      (c) a yth reverse oligonucleotide primer comprising a region with complementarity to a second region of the yth member of the plurality of target nucleic acids;
   wherein, in (c), the xth signal oligonucleotide probe is degraded by the nucleic acid polymerase such that an xth signal is generated if at least one of the yth member of the plurality of target nucleic acids is present in the mixture;
   wherein (e) further comprises correlating the presence of the xth signal to the presence of at least one of the yth members of the plurality target nucleic acids in the sample; and
   wherein x is an integer greater than 1 and y is an integer greater than n.
16. The method of paragraph 15, wherein the yth forward oligonucleotide primer further comprises an additional region with complementarity to the signal oligonucleotide probe.
17. The method of paragraph 15 or 16, wherein the yth forward oligonucleotide primer further comprises an additional region with complementarity to the signal oligonucleotide probe.
18. A kit for detecting the presence or absence of at least one member of a plurality of target nucleic acids in a sample, comprising:
   (a) a signal oligonucleotide probe;
   (b) a first forward oligonucleotide primer comprising: a first region with complementarity to a first member of the plurality of target nucleic acids; and a second region with complementarity or homology to the signal oligonucleotide probe;
   (c) a first reverse oligonucleotide primer comprising a region with complementarity to the first member of the plurality of target nucleic acids;
   (d) a second forward oligonucleotide primer comprising: a first region with complementarity to a second member of the plurality of target nucleic acids; and a second region with complementarity or homology to the signal oligonucleotide probe; and
   (e) a second reverse oligonucleotide primer comprising a region with complementarity to the second member of the plurality of target nucleic acids.
19. A method of detecting the presence or absence of a first and second member of a plurality of target nucleic acids in a sample, the method comprising:
   (a) providing a sample comprising, or potentially comprising, the first and second members of the plurality of target nucleic acids;
   (b) forming a mixture comprising the sample and
      (i) a template nucleic acid;
      (ii) a signal oligonucleotide probe with complementarity to the template nucleic acid;
      (iii) a first oligonucleotide primer comprising a region with complementarity to the first member of the plurality of target nucleic acids; and
      (iv) a second oligonucleotide primer comprising a region with complementarity to the second member of the plurality of target nucleic acids;
   (c) subjecting the mixture to conditions sufficient to anneal the first oligonucleotide primer to the first member of the plurality of target nucleic acids and the second oligonucleotide primer to the second member of the plurality of target nucleic acids, such that the first and second oligonucleotide primers are degraded;
   (d) thermally cycling the mixture under reaction conditions appropriate to amplify the template nucleic acid with polymerase chain reaction (PCR), such that a signal is generated if both the first and second members of the plurality of target nucleic acids are present in the mixture; and
   (e) detecting the presence or absence of the signal.
20. The method of paragraph 19, wherein the first oligonucleotide primer further comprises an additional region with complementarity to a second region of the template nucleic acid.
21. The method of paragraph 19 or 20, wherein the second oligonucleotide primer further comprises an additional region with complementarity to a third region of the template nucleic acid.
22. The method of paragraph 21, wherein, in (c), the additional region of the first oligonucleotide primer and the additional region of the second oligonucleotide primer are released.
23. The method of any of paragraphs 19-22, wherein the mixture further comprises one or more nucleic acid enzymes.
24. The method of any of paragraphs 19-23, wherein the signal oligonucleotide probe comprises a signal tag.
25. The method of any of paragraphs 19-24, further comprising (f) correlating the presence of the signal to the presence of both the first and second members of the plurality of target nucleic acids in the sample.
26. The method of any of paragraphs 19-25, wherein the mixture in (b) further comprises (vi) a third oligonucleotide primer comprising a region with complementarity to a second region of the first member of the plurality of target nucleic acids; and (vii) a fourth oligonucleotide primer comprising a region with complementarity to a second region of the second member of the plurality of target nucleic acids.
27. The method of any of paragraphs 19-26, further comprising, in (c), thermally cycling the mixture under reaction conditions appropriate to amplify the first and second members of the plurality of target nucleic acids with polymerase chain reaction.
28. A method of detecting the presence or absence of a first and second member of a plurality of target nucleic acids in a sample, the method comprising:
   (a) providing a sample comprising, or potentially comprising, the first and second members of the plurality of target nucleic acids
   (b) forming a mixture comprising the sample and
      (i) a template nucleic acid;
      (ii) a signal oligonucleotide probe with complementarity to the template nucleic acid;
      (iii) a first forward oligonucleotide primer comprising a region with complementarity to the first member of the plurality of target nucleic acids;
      (iv) a first reverse oligonucleotide primer comprising a region with complementarity the first member of the plurality of target nucleic acids;
      (v) a second forward oligonucleotide primer comprising a first region with complementarity to the second member of the plurality of target nucleic acids; and
      (vi) a second reverse oligonucleotide primer comprising a region with complementarity to the second member of the plurality of target nucleic acids;
   (c) thermally cycling the mixture under reaction conditions appropriate to amplify the first and second members of the plurality of target nucleic acids with polymerase chain reaction (PCR), such that the first forward oligonucleotide primer and the second forward oligonucleotide primer are degraded;
   (d) thermally cycling the mixture under reaction conditions appropriate to amplify the template nucleic acid with PCR, such that a signal is generated if both the first and second members of the plurality of target nucleic acids are present in the mixture; and
   (e) detecting the presence or absence of the signal.
29. The method of paragraph 28, further comprising (f) correlating the presence of the signal to the presence of both the first and second members of the plurality of target nucleic acids in the sample.
30. The method of paragraph 28 or 29, wherein the signal oligonucleotide probe comprises a signal tag.
31. The method of any of paragraphs 28-30, wherein the first forward oligonucleotide primer further comprises an additional region with complementarity to a second region of the template nucleic acid.
32. The method of paragraph 31, wherein, in (c), the additional region of the first forward oligonucleotide primer is released.
33. The method of any of paragraphs 28-32, wherein the second forward oligonucleotide primer further comprises an additional region with complementarity to a third region of the template nucleic acid.
34. The method of paragraph 33, wherein, in (c), the additional region of the second forward oligonucleotide primer is released.
35. The method of any of paragraphs 28-34, wherein mixture further comprises a nucleic acid enzyme.
36. The method of paragraph 35, wherein, in (c), the first and second oligonucleotide primers are degraded by the nucleic acid enzyme.
37. A kit for detecting the presence or absence of a first and second member of a plurality of target nucleic acids in a sample, comprising:
   (a) a template nucleic acid;
   (b) a signal oligonucleotide probe with complementarity to a first region of the template nucleic acid;
   (c) a first oligonucleotide primer comprising: a first region with complementarity to a region of the first member of the plurality of target nucleic acids; and a second region with complementarity to a second region of the template nucleic acid; and
   (d) a second oligonucleotide primer comprising: a first region with complementarity to a region of the second member of the plurality of target nucleic acids; and a second region with complementarity to a third region of the template nucleic acid.
38. A method of detecting the presence or absence of at least one member of a plurality of target nucleic acids in a sample, the method comprising:
   (a) providing a sample comprising, or potentially comprising, at least one member of the plurality of target nucleic acids;
   (b) forming a mixture of the sample and
      (i) a signal oligonucleotide probe comprising a signal tag;
      (ii) a plurality of a first forward oligonucleotide primer comprising: a first region with complementarity to a first region of a first member of the plurality of target nucleic acids; and a second region with complementarity to the signal oligonucleotide probe;
      (iii) a plurality of a first reverse oligonucleotide primer comprising a region with complementarity to a second region of the first member of the plurality of target nucleic acids;
      (iv) a plurality of a second forward oligonucleotide primer comprising: a first region with complementarity to a first region of a second member of the plurality of target nucleic acids; and a second region with complementarity to the signal oligonucleotide probe;
      (v) a plurality of a second reverse oligonucleotide primer comprising a region with complementarity to a second region of the second member of the plurality of target nucleic acids; and
      (vi) a nucleic acid enzyme;
   (c) thermally cycling the mixture under reaction conditions appropriate to amplify each member of the plurality of target nucleic acids with polymerase chain reaction (PCR), such that the signal oligonucleotide probe is degraded by the nucleic acid enzyme such that a signal is generated if at least one member of the plurality of target nucleic acids is present in the mixture;
   (d) detecting the presence or absence of the signal; and
   (e) correlating the presence of the signal to the presence of at least one of the members of the plurality of target nucleic acids in the sample.
39. The method of paragraph 38, wherein the mixture formed at (b) further comprises:
   (c) a plurality of an nth forward oligonucleotide primer comprising: a first region with complementarity to a first region of an nth member of the plurality of target nucleic acids; and a second region with complementarity to the signal oligonucleotide probe; and
   (d) a plurality of an nth reverse oligonucleotide primer comprising a region with complementarity to a second region of an nth member of the plurality of target nucleic acids;
   wherein n is an integer greater than 2.
40. The method of paragraph 39;
   wherein the mixture formed at (b) further comprises:
      (d) an xth signal oligonucleotide probe comprising an xth signal tag;
      (e) a plurality of a yth forward oligonucleotide primer comprising: a first region with complementarity to a first region of a yth member of the plurality of target nucleic acids; and a second region with complementarity to the xth signal oligonucleotide probe; and
      (f) a plurality of a yth reverse oligonucleotide primer comprising a region with complementarity to a second region of the yth member of the plurality of target nucleic acids;
   wherein, in (c), the xth signal oligonucleotide probe is degraded by the nucleic acid polymerase such that an xth signal is generated if at least one of the yth member of the plurality of target nucleic acids is present in the mixture;
   wherein (e) further comprises correlating the presence of the xth signal to the presence of at least one of the yth members of the plurality target nucleic acids in the sample; and
   wherein x is an integer greater than 1 and y is an integer greater than n.

## Claims

1. A method of detecting the presence or absence of a first and second member of a plurality of target nucleic acids in a sample, the method comprising:
(a) providing a sample comprising, or potentially comprising, the first and second members of the plurality of target nucleic acids;
(b) forming a mixture comprising the sample and
(i) a template nucleic acid;
(ii) a signal oligonucleotide probe with complementarity to the template nucleic acid;
(iii) a first forward oligonucleotide primer comprising a region with complementarity to the first member of the plurality of target nucleic acids; and
(iv) a second forward oligonucleotide primer comprising a region with complementarity to the second member of the plurality of target nucleic acids;
(c) subj ecting the mixture to conditions sufficient to anneal the first oligonucleotide primer to the first member of the plurality of target nucleic acids and the second oligonucleotide primer to the second member of the plurality of target nucleic acids, such that the first and second oligonucleotide primers are degraded;
(d) thermally cycling the mixture under reaction conditions appropriate to amplify the template nucleic acid with polymerase chain reaction (PCR), such that a signal is generated if both the first and second members of the plurality of target nucleic acids are present in the mixture;
(e) detecting the presence or absence of the signal; and
(f) correlating the presence of the signal to the presence of both the first and second members of the plurality of target nucleic acids in the sample.

2. The method of claim 1, wherein the first oligonucleotide primer further comprises an additional region with complementarity to a second region of the template nucleic acid.

3. The method of claim 1 or 2, wherein the second oligonucleotide primer further comprises an additional region with complementarity to a third region of the template nucleic acid.

4. The method of claim 3, wherein, in (c), the additional region of the first oligonucleotide primer and the additional region of the second oligonucleotide primer are released.

5. The method of claim 4, wherein, in (d), the additional regions of the first oligonucleotide primer and the additional regions of the second oligonucleotide primer anneal to the template nucleic acid.

6. The method of any of claims 1-5, wherein the mixture in (b) further comprises (vi) a third oligonucleotide primer comprising a region with complementarity to a second region of the first member of the plurality of target nucleic acids; and (vii) a fourth oligonucleotide primer comprising a region with complementarity to a second region of the second member of the plurality of target nucleic acids.

7. The method of any of claims 1-6, wherein the mixture further comprises:
(v) a first reverse oligonucleotide primer comprising a region with complementarity to the first member of the plurality of target nucleic acids and
(vi) a second reverse oligonucleotide primer comprising a region with complementarity to the second member of the plurality of target nucleic acids;

8. The method of any of claims 1-7, wherein said a signal oligonucleotide probe further comprises a signal tag.

9. The method of claim 8, wherein the signal tag generates the signal upon degradation of the signal oligonucleotide probe.

10. The method of claim 8 or 9, wherein the signal tag comprises a fluorophore and a quencher.

11. The method of any of claims 1-10, wherein, in (c), the first and second oligonucleotide primers are degraded by a nucleic acid enzyme.

12. The method of claim 11, wherein the nucleic acid enzyme comprises RNase activity or is an RNase

13. The method of claim any of claims 1-12, wherein a first forward oligonucleotide primer or the second forward oligonucleotide primer comprises a non-natural nucleotide.

14. A kit for detecting the presence or absence of a first and second member of a plurality of target nucleic acids in a sample, comprising:
(a) a template nucleic acid;
(b) a signal oligonucleotide probe with complementarity to a first region of the template nucleic acid;
(c) a first oligonucleotide primer comprising: a first region with complementarity to a region of the first member of the plurality of target nucleic acids; and a second region with complementarity to a second region of the template nucleic acid; and
(d) a second oligonucleotide primer comprising: a first region with complementarity to a region of the second member of the plurality of target nucleic acids; and a second region with complementarity to a third region of the template nucleic acid.

15. The kit of claim 14, further comprising: (e) a first reverse oligonucleotide primer comprising a region with complementarity the first member of the plurality of target nucleic acids; and (f) a second reverse oligonucleotide primer comprising a region with complementarity to the second member of the plurality of target nucleic acid.
